# EUROPÄISCHE PATENTSCHRIFT

(11) **EP 1 244 634 B1**
(45) Veröffentlichungstag und Bekanntmachung des Hinweises auf die Patenterteilung: **17.03.2004**
(21) Anmeldenummer: 00991172.8
(22) Anmeldetag: 12.12.2000
(51) Int. Cl.: C07D 233/32, C07D 239/10, A01N 43/50, A01N 43/54

(54) **SUBSTITUIERTE BENZOYLCYCLOHEXANDIONE ALS HERBIZIDE**
SUBSTITUTED BENZOYLCYCLOHEXANE DIONES FOR USE AS HERBICIDES
BENZOYLCYCLOHEXANEDIONES SUBSTITUEES UTILISEES COMME HERBICIDES

(30) Priorität: 24.12.1999 DE 19962923
(43) Veröffentlichungstag der Anmeldung: 02.10.2002
(73) Patentinhaber: Bayer CropScience AG, 40789 Monheim (DE)
(72) Erfinder: MÜLLER, Klaus-Helmut, 40593 Düsseldorf (DE); SCHWARZ, Hans-Georg, 40764 Langenfeld (DE); LEHR, Stefan, 40764 Langenfeld (DE); SCHALLNER, Otto, 40789 Monheim (DE); HOISCHEN, Dorothee, 40474 Düsseldorf (DE); DREWES, Mark, Wilhelm, 40764 Langenfeld (DE); DAHMEN, Peter, 41470 Neuss (DE); FEUCHT, Dieter, 40789 Monheim (DE); PONTZEN, Rolf, 42799 Leichlingen (DE)
(86) Internationale Anmeldenummer: PCT/EP2000/012583
(87) Internationale Veröffentlichungsnummer: WO 2001/047894

(56) Entgegenhaltungen:
- EP-A- 0 058 868
- WO-A-94/22826
- WO-A-95/34540
- WO-A-97/46530
- WO-A-99/07688
- DE-A- 19 921 732
- GB-A- 1 327 552
- US-A- 5 852 192
- US-A- 6 004 903
- DATABASE CHEMABS [Online] CHEMICAL ABSTRACTS SERVICE, COLUMBUS, OHIO, US; OGAWA, HIDENORI ET AL: "Preparation and formulation of benzazepine derivatives and analogs as pharmaceuticals with affinity for vasopressin receptors" retrieved from STN Database accession no. 127:248027 XP002162488 & JP 09 221476 A (OTSUKA PHARMACEUTICAL CO., LTD., JAPAN) 26. August 1997 (1997-08-26)
- DATABASE CHEMABS [Online] CHEMICAL ABSTRACTS SERVICE, COLUMBUS, OHIO, US; BABCZINSKI, PETER ET AL: "Substituted tetrahydropyrimidinones: a new herbicidal class of compounds inducing chlorosis by inhibition of phytoene desaturation. 2. Structure-activity relationships" retrieved from STN Database accession no. 123:49747 XP002162489 & PESTIC. BIOCHEM. PHYSIOL. (1995), 52(1), 45-59,
- DATABASE CHEMABS [Online] CHEMICAL ABSTRACTS SERVICE, COLUMBUS, OHIO, US; NAGARAJAN, K. ET AL: "Nitroimidazoles. Part IV. 1-Sulfonyl(carbamoyl/thiocarbamoyl)-3-(1- methyl-5-nitroimidazol-2-yl)-2-imidazolidi nones" retrieved from STN Database accession no. 98:215526 XP002162491 & INDIAN J. CHEM., SECT. B (1982), 21B(10), 928-40,
- DATABASE CHEMABS [Online] CHEMICAL ABSTRACTS SERVICE, COLUMBUS, OHIO, US; CRAM, DONALD J. ET AL: "Strongly binding, rapidly complexing, ion selective spherands" retrieved from STN Database accession no. 98:53855 XP002162492 & J. CHEM. SOC., CHEM. COMMUN. (1982), (21), 1219-21,
- DATABASE CHEMABS [Online] CHEMICAL ABSTRACTS SERVICE, COLUMBUS, OHIO, US; CRAM, D. J. ET AL: "Host-guest complexation. 32. Spherands composed of cyclic urea and anisyl units" retrieved from STN Database accession no. 101:210478 XP002162751 & J. AM. CHEM. SOC. (1984), 106(23), 7150-67 ,

## Beschreibung

Die Erfindung betrifft neue substituierte Benzoylcyclohexandione, Verfahren zu ihrer Herstellung und ihre Verwendung als Herbizide,

Es ist bereits bekannt, dass bestimmte substituierte Benzoylcyclohexandione herbizide Eigenschaften aufweisen (vgl. EP-A-090262, EP-A-135191, EP-A-186118, EP-A-186119, EP-A-186120, EP-A-319075, DE-A-199 21 732, WO-A-96/26200, WO-A-97/46530, WO-A-99/07688). Die Wirkung dieser Verbindungen ist jedoch nicht in allen Belangen zufriedenstellend.

Weitere substituierte Benzoylcyclohexandione sind Gegenstand einer vorgängigen, jedoch nicht vorveröffentlichten Patentanmeldung (vgl. DE-A 19 921 732).

Es wurden nun die neuen substituierten Benzoylcyclohexandione der allgemeinen Formel (I) gefunden, 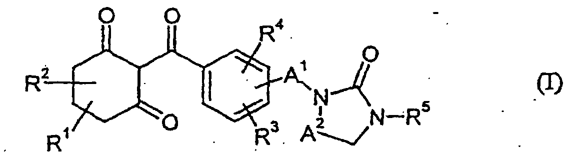 in welcher
- A¹: für Alkandiyl (Alkylen) mit 1 bis 3 Kohlenstoffatomen steht,
- A²: für Alkandiyl (Alkylen) mit 1 bis 3 Kohlenstoffatomen steht,
- R¹: für Wasserstoff, für Phenyl oder für jeweils gegebenenfalls durch Halogen substituiertes Alkyl, Alkylthio oder Alkoxycarbonyl mit jeweils 1 bis 4 Kohlenstoffatomen in den Alkylgruppen steht,
- R²: für Wasserstoff oder für jeweils gegebenenfalls durch Halogen substituiertes Alkyl oder Alkylthio mit jeweils 1 bis 4 Kohlenstoffatomen steht, oder zusammen mit R¹ für Alkandiyl (Alkylen) mit bis zu 4 Kohlenstoffatomen steht, oder - für den Fall, dass R¹ und R² an das gleiche Kohlenstoffatom gebunden sind - zusammen mit R¹ und dem Kohlenstoffatom, an das R¹ und R² gebunden sind, für eine Carbonyl-Gruppierung (C=O) steht,
- R³: für Wasserstoff, Nitro, Cyano, Carboxy, Carbamoyl, Thiocarbamoyl, Halogen, für jeweils gegebenenfalls durch Halogen substituiertes Alkyl, Alkoxy, Alkylthio, Alkylsulfinyl, Alkylsulfonyl, Alkylamino, Dialkylamino, Alkylsulfonylamino oder Dialkylaminosulfonyl mit jeweils 1 bis 4 Kohlenstoffatomen in den Alkylgruppen, oder für jeweils gegebenenfalls durch Halogen oder C₁-C₄-Alkyl substituiertes Cycloalkylamino, Cycloalkylsulfonyl, Cycloallcylsulfonylamino oder Cycloalkylaminosulfonyl mit jeweils 3 bis 6 Kohlenstoffatomen steht,
- R⁴: für Wasserstoff, Nitro, Cyano, Carboxy, Carbamoyl, Thiocarbamoyl, Halogen, oder für jeweils gegebenenfalls durch Halogen substituiertes Alkyl, Alkoxy, Alkylthio, Alkylsulfinyl, Alkylsulfonyl, Alkylamino, Dialkylamino oder Dialkylaminosulfonyl mit jeweils 1 bis 4 Kohlenstoffatomen in den Alkylgruppen steht, und
- R⁵: für Wasserstoff, für Amino, für jeweils gegebenenfalls durch Amino, Cyano, Halogen oder C₁-C₄-Alkoxy substituiertes Alkyl, Alkoxy, Alkylamino, Dialkylamino, Alkylsulfonyl oder Alkylsulfonylamino mit jeweils 1 bis 5 Kohlenstoffatomen in den Alkylgruppen, für jeweils gegebenenfalls durch Halogen substituiertes Alkenyl oder Alkinyl mit jeweils bis zu 5 Kohlenstoffatomen, für jeweils gegebenenfalls durch Cyano, Halogen oder C₁-C₄-Alkyl substituiertes Cycloalkyl oder Cycloalkylalkyl mit jeweils 3 bis 6 Kohlenstoffatomen in der Cycloalkylgruppe und gegebenenfalls 1 bis 3 Kohlenstoffatomen im Alkylteil, für Cycloalkenyl mit 5 oder 6 Kohlenstoffatomen, oder für jeweils gegebenenfalls durch Nitro, Cyano, Carboxy, Halogen, C₁-C₄-Alkyl, C₁-C₄-Halogenalkyl, C₁-C₄-Alkoxy oder C₁-C₄-Halogenalkoxy substituiertes Phenyl, Phenyl-C₁-C₄-alkyl, Pyridinyl oder Pyrimidinyl steht,
wobei jedoch die Verbindung 1-[2,6-Dichlor-3-[(2-hydroxy-6-oxo-cyclohexen-1-yl)-carbonyl]-benzyl]-3-methyl-tetrahydro-2(1H)-pyrimidinon (vgl. DE-A 19 921 732, Tabelle 2, Beispiel ID-1) ausgenommen ist.

Gegenstand der Erfindung sind auch alle möglichen tautomeren Formen der Verbindungen der allgemeinen Formel (I) und die möglichen Salze bzw. Metallkomplexe der Verbindungen der allgemeinen Formel (I).

In den Definitionen sind die Kohlenwasserstoffketten, wie Alkyl oder Alkandiyl - auch in Verbindung mit Heteroatomen, wie in Alkoxy - jeweils geradkettig oder verzweigt.

Neben den Verbindungen der allgemeinen Formel (I) - oben - können immer auch die entsprechenden tautomeren Formen - nachstehend beispielhaft dargestellt - vorliegen.

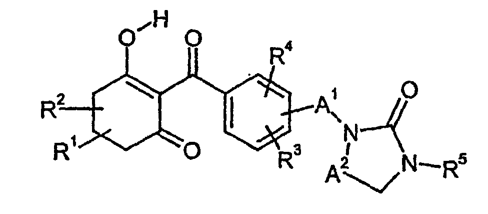

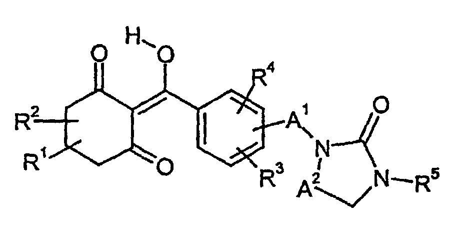

Bevorzugte Bedeutungen der in den vorstehend gezeigten Formeln aufgeführten Reste / Substituenten werden im Folgenden definiert.
- A¹: steht bevorzugt, für Methylen, Ethan-1,1-diyl (Ethyliden), Ethan-1,2-diyl (Dimethylen), Propan-1,1-diyl, Propan-1,2-diyl oder Propan-1,3-diyl.
- A²: steht bevorzugt für Methylen, Ethan-1,1-diyl (Ethyliden), Ethan-1,2-diyl (Dimethylen), Propan-1,1-diyl, Propan-1,2-diyl oder Propan-1,3-diyl.
- R¹: steht bevorzugt für Wasserstoff, für Phenyl oder für jeweils gegebenenfalls durch Fluor und/oder Chlor substituiertes Methyl, Ethyl, n- oder i-Propyl, Methylthio, Ethylthio, n- oder i-Propylthio, Methoxycarbonyl, Ethoxycarbonyl, n- oder i-Propoxycarbonyl.
- R²: steht bevorzugt für Wasserstoff oder für jeweils gegebenenfalls durch Fluor und/oder Chlor substituiertes Methyl, Ethyl, n- oder i-Propyl, Methylthio, Ethylthio, n- oder i-Propylthio, oder zusammen mit R¹ für Methylen, Ethan-1,1-diyl, Ethan-1,2-diyl (Dimethylen), Propan-1,1-diyl, Propan-1,2-diyl oder Propan-1,3-diyl, oder - für den Fall, dass R¹ und R² an das gleiche Kohlenstoffatom gebunden sind - zusammen mit R¹ und dem Kohlenstoffatom, an das R¹ und R² gebunden sind, für eine Carbonyl-Gruppierung (C=O).
- R³: steht bevorzugt für Wasserstoff, Nitro, Cyano, Carboxy, Carbamoyl, Thiocarbamoyl, Fluor, Chlor, Brom, Iod, für jeweils gegebenenfalls durch Fluor und/oder Chlor substituiertes Methyl, Ethyl, n- oder i-Propyl, Methoxy, Ethoxy, n- oder i-Propoxy, Methylthio, Ethylthio, n- oder i-Propylthio, Methylsulfinyl, Ethylsulfinyl, n- oder i-Propylsulfinyl, Methylsulfonyl, Ethylsulfonyl, n- oder i-Propylsulfonyl, für Methylamino, Ethylamino, n- oder i-Propylamino, Dimethylamino, Diethylamino, Methylsulfonylamino, Ethylsulfonylamino, n- oder i-Propylsulfonylamino, Dimethylaminosulfonyl oder Diethylaminosulfonyl, oder für jeweils gegebenenfalls durch Fluor, Chlor oder Methyl substituiertes Cyclopropylamino, Cyclobutylamino, Cyclopentylamino, Cyclohexylamino, Cyclopropylsulfonyl, Cyclobutylsulfonyl, Cyclopentylsulfonyl, Cyclohexylsulfonyl, Cyclopropylsulfonylamino, Cyclobutylsulfonylamino, Cyclopentylsulfonylamino, Cyclohexylsulfonylamino, Cyclopropylaminosulfonyl, Cyclobuiylaminosulfonyl, Cyclopentylaminosulfonyl oder Cyclohexylaminosulfonyl.
- R⁴: steht bevorzugt für Wasserstoff, Nitro, Cyano, Carboxy, Carbamoyl, Thiocarbamoyl, Fluor, Chlor, Brom, Iod, oder für jeweils gegebenenfalls durch Fluor und/oder Chlor substituiertes Methyl, Ethyl, n- oder i-Propyl, Methoxy, Ethoxy, n- oder i-Propoxy, Methylthio, Ethylthio, n- oder i-Propylthio, Methylsulfinyl, Ethylsulfinyl, n- oder i-Propylsulfinyl, Methylsulfonyl, Ethylsulfonyl, n- oder i-Propylsulfonyl, für Methylamino, Ethylamino, n- oder i-Propylamino, Dimethylamino, Diethylamino, Dimethylaminosulfonyl oder Diethylaminosulfonyl.
- R⁵: steht bevorzugt für Wasserstoff, für Amino, für jeweils gegebenenfalls durch Amino, Cyano, Fluor und/oder Chlor, Methoxy oder Ethoxy substituiertes Methyl, Ethyl, n- oder i-Propyl, n-, i-, s- oder t-Butyl, Methoxy, Ethoxy, n- oder i-Propoxy, n-, i-, s- oder t-Butoxy, Methylamino, Ethylamino, n- oder iPropylamino, n-, i-, s- oder t-Butylamino, Dimethylamino, Diethylamino, Dipropylamino, Methylsulfonyl, Ethylsulfonyl oder Methylsulfonylamino, für jeweils gegebenenfalls durch Fluor, Chlor und/oder Brom substituiertes Propenyl, Butenyl, Propinyl oder Butinyl, für jeweils gegebenenfalls durch Cyano, Fluor, Chlor, Methyl oder Ethyl substituiertes Cyclopropyl, Cyclobutyl, Cyclopentyl, Cyclohexyl, Cyclopropylmethyl, Cyclobutylmethyl, Cyclopentylmethyl oder Cyclohexylmethyl, für Cyclohexenyl, oder für jeweils gegebenenfalls durch Fluor, Chlor, Brom, Methyl, Trifluormethyl, Methoxy, Difluormethoxy oder Trifluormethoxy substituiertes Phenyl, Benzyl, Pyridinyl oder Pyrimidinyl.
- A¹: steht besonders bevorzugt für Methylen, Ethan-1,2-diyl (Dimethylen) oder Propan-1,3-diyl.
- A²: steht besonders bevorzugt für Methylen, Ethan-1,2-diyl (Dimethylen) oder Propan-1,3-diyl.
- R¹: steht besonders bevorzugt für Wasserstoff, Phenyl, Methyl, Ethyl, n- oder i-Propyl, Methylthio, Ethylthio, Methoxycarbonyl oder Ethoxycarbonyl.
- R²: steht besonders bevorzugt für Wasserstoff, Methyl, Ethyl, Methylthio, Ethylthio, oder zusammen mit R¹ für Methylen, Ethan-1,2-diyl (Dimethylen) oder Propan-1,3-diyl (Trimethylen), oder - für den Fall, dass R¹ und R² an das gleiche Kohlenstoffatom gebunden sind - zusammen mit R¹ und dem Kohlenstoffatom, an das R¹ und R² gebunden sind, für eine Carbonyl-Gruppierung (C=O).
- R³: steht besonders bevorzugt für Wasserstoff, Nitro, Cyano, Fluor, Chlor, Brom, Iod, für jeweils gegebenenfalls durch Fluor und/oder Chlor substituiertes Methyl, Ethyl, Methoxy, Ethoxy, Methylthio, Ethylthio, Methylsulfinyl, Ethylsulfinyl, Methylsulfonyl, Ethylsulfonyl, für Methylamino, Ethylamino, Dimethylamino oder Dimethylaminosulfonyl.
- R⁴: steht besonders bevorzugt für Wasserstoff, Nitro, Cyano, Fluor, Chlor, Brom, Iod, für jeweils gegebenenfalls durch Fluor und/oder Chlor substituiertes Methyl, Ethyl, Methoxy, Ethoxy, Methylthio, Ethylthio, Methylsulfinyl, Ethylsulfinyl, Methylsulfonyl, Ethylsulfonyl, für Methylamino, Ethylamino, Dimethylamino oder Dimethylaminosulfonyl.

- R⁵: steht besonders bevorzugt für Wasserstoff, Methyl, Ethyl, n- oder i-Propyl, n-, i-, s- oder t-Butyl, Propenyl, Propinyl, Cyclopropyl, Cyclopentyl, Cyclohexyl, Cyclopropylmethyl, Cyclopentylmethyl, Cyclohexylmethyl, Phenyl oder Benzyl.
- A¹: steht ganz besonders bevorzugt für Methylen oder Ethan-1,2-diyl (Dimethylen).
- A²: steht ganz besonders bevorzugt für Methylen oder Etnan-1,2-diyl (Dimethylen).
- R¹: steht ganz besonders bevorzugt für Wasserstoff, Phenyl, Methyl, Ethyl, n-oder i-Propyl, Methylthio oder Ethylthio.
- R²: steht ganz besonders bevorzugt für Wasserstoff, Methyl, Ethyl, Methylthio, oder zusammen mit R¹ für Ethan-1,2-diyl oder Propan-1,3-diyl, oder - für den Fall, dass R¹ und R² an das gleiche Kohlenstoffatom gebunden sind - zusammen mit R¹ und dem Kohlenstoffatom, an das R¹ und R² gebunden sind, für eine Carbonyl-Gruppierung (C=O).
- R³: steht ganz besonders bevorzugt für Wasserstoff, Nitro, Cyano, Fluor, Chlor, Brom, Methyl, Difluormethyl, Trifluormethyl, Dichlormethyl, Trichlormethyl, Chlordifluormethyl, Ethyl, Methoxy, Difluormethoxy, Trifluormethoxy, Chlordifluormethoxy, Ethoxy, Methylthio, Difluormethylthio, Trifluormethylthio, Chlordifluormethylthio, Ethylthio, Methylsulfinyl, Trifluormethylsulfinyl, Ethylsulfinyl, Methylsulfonyl, Trifluormethylsulfonyl, Ethylsulfonyl, Dimethylamino oder Dimethylaminosulfonyl.
- R⁴: steht ganz besonders bevorzugt für Wasserstoff, Nitro, Cyano, Fluor, Chlor, Brom, Methyl, Difluormethyl, Trifluormethyl, Dichlormethyl, Trichlormethyl, Chlordifluormethyl, Ethyl, Methoxy, Difluormethoxy, Trifluormethoxy, Chlordifluormethoxy, Ethoxy, Methylthio, Difluormethylthio, Trifluormethylthio, Chlordifluormethylthio, Ethylthio, Methylsulfinyl, Trifluormethylsulfinyl, Ethylsulfinyl, Methylsulfonyl, Trifluormethylsulfonyl, Ethylsulfonyl, Dimethylamino oder Dimethylaminosulfonyl.
- R⁵: steht ganz besonders bevorzugt für Wasserstoff, Methyl, Ethyl, n- oder i-Propyl, n-, i-, s- oder t-Butyl, Cyclopropyl, Cyclopentyl oder Cyclohexyl.

Gegenstand der Erfindung sind vorzugsweise auch Natrium-, Kalium-, Magnesium-, Calcium-, Ammonium-, C₁-C₄-Alkyl-ammonium-, Di-(C₁-C₄-alkyl)-ammonium-, Tri-(C₁-C₄-alkyl)-ammonium-, Tetra-(C₁-C₄-alkyl)-ammonium, Tri-(C₁-C₄-alkyl)-sulfonium-, C₅- oder C₆-Cycloalkyl-ammonium- und Di-(C₁-C₂-alkyl)-benzylammonium-Salze von Verbindungen der Formel (I), in welcher A¹, A², R¹, R², R³, R⁴ und R⁵ die oben angegebenen Bedeutungen haben, oder auch Komplexverbindungen (Koordinationsverbindungen) dieser Verbindungen mit Metallen wie Kupfer, Eisen, Kobalt, Nickel.

Erfindungsgemäß bevorzugt sind diejenigen Verbindungen der Formel (I), bei welchen eine Kombination der vorstehend als bevorzugt aufgeführten Bedeutungen vorliegt.

Erfindungsgemäß besonders bevorzugt sind diejenigen Verbindungen der Formel (I), bei welchen eine Kombination der vorstehend als besonders bevorzugt aufgeführten Bedeutungen vorliegt.

Erfindungsgemäß ganz besonders bevorzugt sind diejenigen Verbindungen der Formel (I), bei welchen eine Kombination der vorstehend als ganz besonders bevorzugt aufgeführten Bedeutungen vorliegt.

Die oben aufgeführten allgemeinen oder in Vorzugsbereichen aufgeführten Restedefinitionen gelten sowohl für die Endprodukte der Formel (I) als auch entsprechend für die jeweils zur Herstellung benötigten Ausgangs- oder Zwischenprodukte. Diese Restedefinitionen können untereinander, also auch zwischen den angegebenen bevorzugten Bereichen beliebig kombiniert werden.

Verbindungen der folgenden allgemeinen Formeln (IA), (IB) und (IC) - wobei A¹, A², R¹, R², R³ und R⁴ die oben als ganz besonders bevorzugt angegebene Bedeutung haben - werden insbesondere als erfindungsgemäß hervorgehoben:

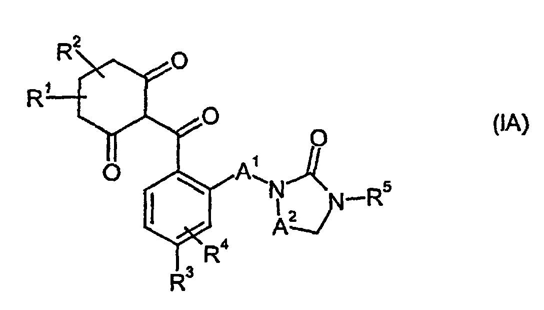

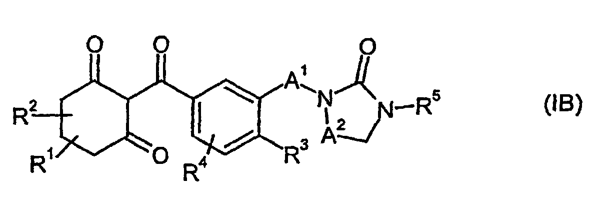

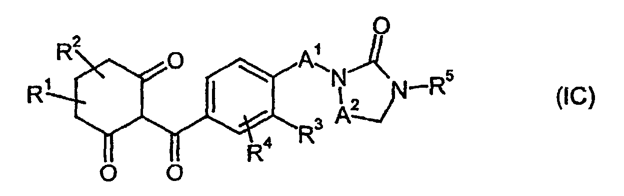

Die neuen substituierten Benzoylcyclohexandione der allgemeinen Formel (I) zeichnen sich durch starke und selektive herbizide Wirksamkeit aus.

Man erhält die neuen substituierten Benzoylcyclohexandione der allgemeinen Formel (I), wenn man 1,3-Cyclohexandion oder dessen Derivate der allgemeinen Formel (II), 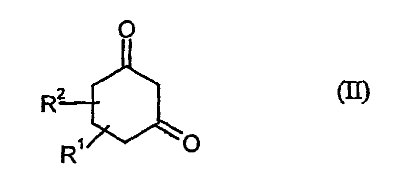 in welcher
- R¹ und R²: die oben angegebene Bedeutung haben,
mit substituierten Benzoesäuren der allgemeinen Formel (III), 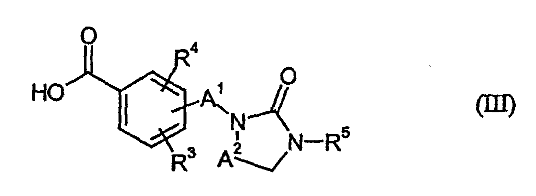 in welcher
- A¹, A², R³, R⁴ und R⁵: die oben angegebene Bedeutung haben,
in Gegenwart eines Dehydratisierungsmittels, gegebenenfalls in Gegenwart eines oder mehrerer Reaktionshilfsmittel und gegebenenfalls in Gegenwart eines Verdünnungsmittels, umsetzt.

Die Verbindungen der allgemeinen Formel (I) können prinzipiell auch wie im Folgenden schematisch dargestellt synthetisiert werden:

Umsetzung von 1,3-Cyclohexandion oder dessen Derivaten der allgemeinen Formel (II) - oben - mit reaktiven Derivaten der substituierten Benzoesäuren der allgemeinen Formel (III) - oben - insbesondere mit entsprechenden Carbonsäure-chloriden, Carbonsäure-anhydriden, Carbonsäure-cyaniden, Carbonsäure-imidazoliden, Carbonsäure-triazoliden, Carbonsäure-methylestern oder -ethylestern - gegebenenfalls in Gegenwart von Reaktionshilfsmitteln, wie z.B. Triethylamin (und gegebenenfalls zusätzlich Zinkchlorid), und gegebenenfalls in Gegenwart eines Verdünnungsmittels, wie z.B. Methylenchlorid: 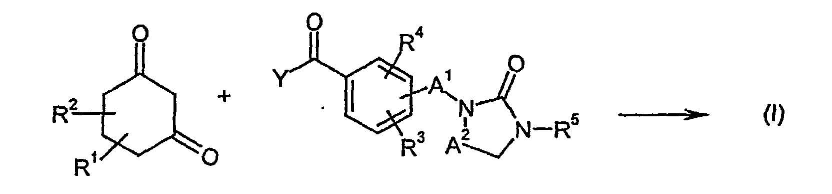 (Y z.B. CN, Cl, Imidazolyl, Triazolyl, Methoxy, Ethoxy)

Bei den oben skizzierten Umsetzungen zur Herstellung der Verbindungen der allgemeinen Formel (I) kommt es im allgemeinen neben der erwünschten C-Benzoylierung am Cyclohexandion auch zu einer O-Benzoylierung - vgl. nachstehendes Formelschema (vgl. Synthesis 1978, 925-927; Tetrahedron Lett. 37 (1996), 1007-1009, WO-A-91/05469). Die hierbei gebildeten O-Benzoyl-Verbindungen werden jedoch unter den Reaktionsbedingungen des erfindungsgemäßen Verfahrens zu den entsprechenden C-Benzoyl-Verbindungen der Formel (I) isomerisiert. 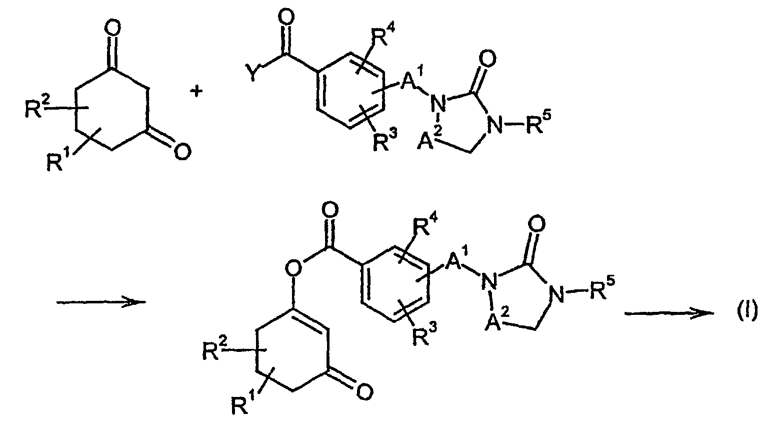 Verwendet man beispielsweise 1,3-Cyclohexandion und 3-Fluor-5-[(3-methyl-2-oxoimidazolidin-1-yl)-methyl]-benzoesäure als Ausgangsstoffe, so kann der Reaktionsablauf beim erfindungsgemäßen Verfahren durch das folgende Formelschema skizziert werden:

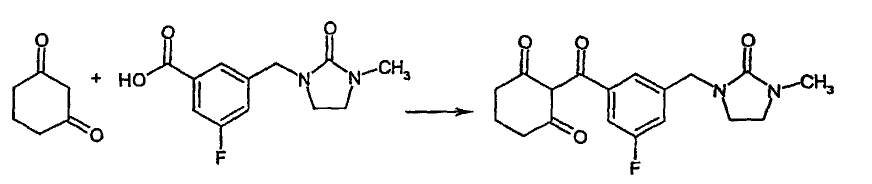

Die beim erfindungsgemäßen Verfahren zur Herstellung von Verbindungen der allgemeinen Formel (I) als Ausgangsstoffe zu verwendenden Cyclohexandione sind durch die Formel (II) allgemein definiert. In der allgemeinen Formel (II) haben R¹ und R² vorzugsweise bzw. insbesondere diejenigen Bedeutungen, die bereits oben im Zusammenhang mit der Beschreibung der erfindungsgemäßen Verbindungen der allgemeinen Formel (I) vorzugsweise bzw. als insbesondere bevorzugt für R¹ und R² angegeben worden sind.

Die Ausgangsstoffe der allgemeinen Formel (II) sind bekannt und/oder können nach an sich bekannten Verfahren hergestellt werden.

Die beim erfindungsgemäßen Verfahren zur Herstellung von Verbindungen der allgemeinen Formel (I) weiter als Ausgangsstoffe zu verwendenden substituierten Benzoesäuren sind durch die Formel (III) allgemein definiert. In der allgemeinen Formel (III) haben A¹, A², R³, R⁴ und R⁵ vorzugsweise diejenigen Bedeutungen, die bereits oben im Zusammenhang mit der Beschreibung der erfindungsgemäßen Verbindungen der allgemeinen Formel (I) als bevorzugt, besonders bevorzugt oder ganz besonders bevorzugt für A¹, A², R³, R⁴ und R⁵ angegeben worden sind.

Die Ausgangsstoffe der allgemeinen Formel (III) sind neu, können jedoch nach an sich bekannten Verfahren hergestellt werden (vgl. US-A 3 833 586, WO-A-94/22826, WO-A-95/34540). Die Verbindungen der Formel (III) sind deshalb ebenfalls Gegenstand der vorliegenden Erfindung.

Man erhält die substituierten Benzoesäuren der allgemeinen Formel (III), wenn man entsprechende substituierte Benzoesäureester der allgemeinen Formel (IV) 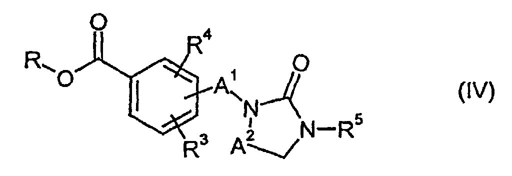 in welcher
- A¹, A², R³, R⁴ und R⁵: die oben angegebene Bedeutung haben und
- R: für Alkyl (insbesondere Methyl oder Ethyl) steht,
mit Wasser, gegebenenfalls in Gegenwart eines Hydrolysehilfsmittels, wie z.B. Natriumhydroxid, und gegebenenfalls in Gegenwart eines organischen Lösungsmittels, wie z.B. 1,4-Dioxan, bei Temperaturen zwischen 10°C und 100°C umsetzt (vgl. die Herstellungsbeispiele).

Die als Vorprodukte benötigten substituierten Benzoesäureester der allgemeinen Formel (IV) sind neu, können jedoch nach an sich bekannten Verfahren hergestellt werden (vgl. US-A 3 833 586, WO-A-94/22826, WO-A-95/34540). Die Verbindungen der Formel (IV) sind deshalb ebenfalls Gegenstand der vorliegenden Erfindung.

Man erhält die substituierten Benzoesäureester der allgemeinen Formel (IV), wenn man entsprechende Halogenalkylbenzoesäureester der allgemeinen Formel (V) 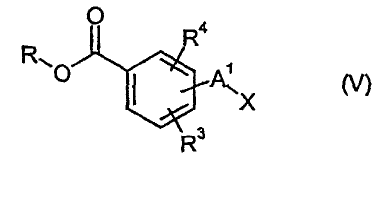 in welcher
- A¹, R, R³ und R⁴: die oben angegebene Bedeutung haben und
- X: für Halogen (insbesondere Fluor, Chlor oder Brom) oder Alkylsulfonyloxy (insbesondere Methylsulfonyloxy oder Ethylsulfonyloxy) steht,
mit 1,3-Diaza-2-oxo-cycloalkanen der allgemeinen Formel (VI) 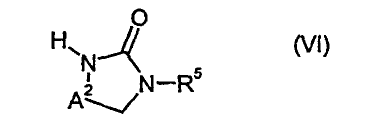 in welcher
- A² und R⁵: die oben angegebene Bedeutung haben,
gegebenenfalls in Gegenwart eines Säureakzeptors, wie z.B. Kaliumcarbonat oder Natriumcarbonat, gegebenenfalls in Gegenwart eines weiteren Reaktionshilfsmittels, wie z.B. Kaliumiodid oder Natriumiodid, in Gegenwart eines Verdünnungsmittels, wie z.B. Aceton, Acetonitril oder N,N-Dimethyl-formamid, bei Temperaturen zwischen 10°C und 120°C umsetzt (vgl. die Herstellungsbeispiele).

Die Verbindungen der Formel (VI) sind teilweise bekannt (J. Amer. Chem. Soc. 78, 5349, (1956), US 2 518 264, Experientia 13, 183 (1957), Chem. Pharm. Bull. 1980, 1810, J. Chem. Soc. Perkin Trans 1, 1998, 423, US 5 972 431) und können wie dort beschrieben hergestellt werden. Einige Verbindungen der Formel (VI) sind jedoch neu (siehe auch Tabelle 5) und sind Gegenstand dieser Anmeldung.

Das erfindungsgemäße Verfahren zur Herstellung der neuen substituierten Benzoylcyclohexandione der allgemeinen Formel (I) wird unter Verwendung eines Dehydratisierungsmittels durchgeführt. Es kommen hierbei die üblichen zur Bindung von Wasser geeigneten Chemikalien in Betracht.

Als Beispiele hierfür werden Dicyclohexylcarbodiimid und Carbonyl-bis-imidazol genannt.

Als besonders gut geeignetes Dehydratisierungsmittel sei Dicyclohexylcarbodiimid genannt.

Das erfindungsgemäße Verfahren zur Herstellung der neuen substituierten Benzoylcyclohexandione der allgemeinen Formel (I) wird gegebenenfalls unter Verwendung eines Reaktionshilfsmittels durchgeführt.

Als Beispiele hierfür werden Natriumcyanid, Kaliumcyanid, Acetoncyanhydrin, 2-Cyano-2-(trimethylsilyloxy)-propan, Trimethylsilylcyanid und 1,2,4-Triazol genannt.

Als besonders gut geeignetes weiteres Reaktionshilfsmittel wird Trimethylsilylcyanid genannt.

Das erfindungsgemäße Verfahren zur Herstellung der neuen substituierten Benzoylcyclohexandione der allgemeinen Formel (I) wird gegebenenfalls unter Verwendung eines weiteren Reaktionshilfsmittels durchgeführt. Als weitere Reaktionshilfsmittel für das erfindungsgemäße Verfahren kommen im allgemeinen basische organische Stickstoffverbindungen, wie beispielsweise Trimethylamin, Triethylamin, Tripropylamin, Tributylamin, Ethyl-diisopropylamin, N,N-Dimethyl-cyclohexylamin, Dicyclohexylamin, Ethyl-dicyclohexylamin, N,N-Dimethyl-anilin, N,N-Dimethylbenzylamin, Pyridin, 2-Methyl-, 3-Methyl-, 4-Methyl-, 2,4-Dimethyl-, 2,6-Dimethyl-, 3,4-Dimethyl- und 3,5-Dimethyl-pyridin, 5-Ethyl-2-methyl-pyridin, 4-Dimethylamino-pyridin, N-Methyl-piperidin, 1,4-Diazabicyclo[2,2,2]-octan (DABCO), 1,5-Diazabicyclo[4,3,0]-non-5-en (DBN), oder 1,8-Diazabicyclo[5,4,0]-undec-7-en (DBU) in Betracht.

Als Verdünnungsmittel zur Durchführung des erfindungsgemäßen Verfahrens kommen vor allem inerte organische Lösungsmittel in Betracht. Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Dichlorbenzol, Petrolether, Hexan, Cyclohexan, Dichlormethan, Chloroform, Tetrachlormethan oder 1,2-Dichlor-ethan, Ether, wie Diethylether, Diisopropylether, Dioxan, Tetrahydrofuran, Ethylenglykoldimethyl- oder -diethylether; Ketone, wie Aceton, Butanon oder Methyl-isobutyl-keton; Nitrile, wie Acetonitril, Propionitril oder Butyronitril; Amide, wie N,N-Dimethylformamid, N,N-Dimethylacetamidy N-Methyl-formanilid, N-Methyl-pyrrolidon oder Hexamethylphosphorsäuretriamid; Ester wie Essigsäuremethylester oder Essigsäureethylester, Sulfoxide, wie Dimethylsulfoxid.

Die Reaktionstemperaturen können bei der Durchführung des erfindungsgemäßen Verfahrens in einem größeren Bereich variiert werden. Im allgemeinen arbeitet man bei Temperaturen zwischen 0°C und 150°C, vorzugsweise zwischen 10°C und 120°C.

Das erfindungsgemäße Verfahren wird im allgemeinen unter Normaldruck durchgeführt. Es ist jedoch auch möglich, das erfindungsgemäße Verfahren unter erhöhtem oder vermindertem Druck - im allgemeinen zwischen 0,1 bar und 10 bar - durchzuführen.

Zur Durchführung des erfindungsgemäßen Verfahrens werden die Ausgangsstoffe im allgemeinen in angenähert äquimolaren Mengen eingesetzt. Es ist jedoch auch möglich, eine der Komponenten in einem größeren Überschuß zu verwenden. Die Umsetzung wird im allgemeinen in einem geeigneten Verdünnungsmittel in Gegenwart eines Dehydratisierungsmittels durchgeführt und das Reaktionsgemisch wird im allgemeinen mehrere Stunden bei der erforderlichen Temperatur gerührt. Die Aufarbeitung wird nach üblichen Methoden durchgeführt (vgl. die Herstellungsbeispiele).

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab. Die erfindungsgemäßen Wirkstoffe können z.B. bei den folgenden Pflanzen verwendet werden:

Dikotyle Unkräuter der Gattungen: Abutilon, Amaranthus, Ambrosia, Anoda, Anthemis, Aphanes, Atriplex, Bellis, Bidens, Capsella, Carduus, Cassia, Centaurea, Chenopodium, Cirsium, Convolvulus, Datura, Desmodium, Emex, Erysimum, Euphorbia, Galeopsis, Galinsoga, Galium, Hibiscus, Ipomoea, Kochia, Lamium, Lepidium, Lindernia, Matricaria, Mentha, Mercurialis, Mullugo, Myosotis, Papaver, Pharbitis, Plantago, Polygonum, Portulaca, Ranunculus, Raphanus, Rorippa, Rotala, Rumex, Salsola, Senecio, Sesbania, Sida, Sinapis, Solanum, Sonchus, Sphenoclea, Stellaria, Taraxacum, Thlaspi, Trifolium, Urtica, Veronica, Viola, Xanthium.

Dikotyle Kulturen der Gattungen: Arachis, Beta, Brassica, Cucumis, Cucurbita, Helianthus, Daucus, Glycine, Gossypium, Ipomoea, Lactuca, Linum, Lycopersicon, Nicotiana, Phaseolus, Pisum, Solanum, Vicia.

Monokotyle Unkräuter der Gattungen: Aegilops, Agropyron, Agrostis, Alopecurus, Apera, Avena, Brachiaria, Bromus, Cenchrus, Commelina, Cynodon, Cyperus, Dactyloctenium, Digitaria, Echinochloa, Eleocharis, Eleusine, Eragrostis, Eriochloa, Festuca, Fimbristylis, Heteranthera, Imperata, Ischaemum, Leptochloa, Lolium, Monochoria, Panicum, Paspalum, Phalaris, Phleum, Poa, Rottboellia, Sagittaria, Scirpus, Setaria, Sorghum.

Monokotyle Kulturen der Gattungen: Allium, Ananas, Asparagus, Avena, Hordeum, Oryza, Panicum, Saccharum, Secale, Sorghum, Triticale, Triticum, Zea.

Die Verwendung der erfindungsgemäßen Wirkstoffe ist jedoch keineswegs auf diese Gattungen beschränkt, sondern erstreckt sich in gleicher Weise auch auf andere Pflanzen.

Die erfindungsgemäßen Wirkstoffe eignen sich in Abhängigkeit von der Konzentration zur Totalunkrautbekämpfung, z.B. auf Industrie- und Gleisanlagen und auf Wegen und Plätzen mit und ohne Baumbewuchs. Ebenso können die erfindungsgemäßen Wirkstoffe zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht- und Hopfenanlagen, auf Zier- und Sportrasen und Weideflächen sowie zur selektiven Unkrautbekämpfung in einjährigen Kulturen eingesetzt werden.

Die erfindungsgemäßen Verbindungen der Formel (I) zeigen starke herbizide Wirksamkeit und ein breites Wirkungsspektrum bei Anwendung auf dem Boden und auf oberirdische Pflanzenteile. Sie eignen sich in gewissem Umfang auch zur selektiven Bekämpfung von monokotylen und dikotylen Unkräutern in monokotylen und dikotylen Kulturen, sowohl im Vorauflauf- als auch im Nachauflauf-Verfahren.

Die erfindungsgemäßen Wirkstoffe können in bestimmten Konzentrationen bzw. Aufwandmengen auch zur Bekämpfung von tierischen Schädlingen und pilzlichen oder bakteriellen Pflanzenkrankheiten verwendet werden. Sie lassen sich gegebenenfalls auch als Zwischen- oder Vorprodukte für die Synthese weiterer Wirkstoffe einsetzen.

Erfindungsgemäß können alle Pflanzen und Pflanzenteile behandelt werden. Unter Pflanzen werden hierbei alle Pflanzen und Pflanzenpopulationen verstanden, wie erwünschte und unerwünschte Wildpflanzen oder Kulturpflanzen (einschließlich natürlich vorkommender Kulturpflanzen). Kulturpflanzen können Pflanzen sein, die durch konventionelle Züchtungs- und Optimierungsmethoden oder durch biotechnologische und gentechnologische Methoden oder Kombinationen dieser Methoden erhalten werden können, einschließlich der transgenen Pflanzen und einschließlich der durch Sortenschutzrechte schützbaren oder nicht schützbaren Pflanzensorten. Unter Pflanzenteilen sollen alle oberirdischen und unterirdischen Teile und Organe der Pflanzen, wie Sproß, Blatt, Blüte und Wurzel verstanden werden, wobei beispielhaft Blätter, Nadeln, Stengel, Stämme, Blüten, Fruchtkörper, Früchte und Samen sowie Wurzeln, Knollen und Rhizome aufgeführt werden. Zu den Pflanzenteilen gehört auch Erntegut sowie vegetatives und generatives Vermehrungsmaterial, beispielsweise Stecklinge, Knollen, Rhizome, Ableger und Samen.

Die erfindungsgemäße Behandlung der Pflanzen und Pflanzenteile mit den Wirkstoffen erfolgt direkt oder durch Einwirkung auf deren Umgebung, Lebensraum oder Lagerraum nach den üblichen Behandlungsmethoden, z.B. durch Tauchen, Sprühen, Verdampfen, Vernebeln, Streuen, Aufstreichen und bei Vermehrungsmaterial, insbesondere bei Samen, weiterhin durch ein- oder mehrschichtiges Umhüllen.

Die Wirkstoffe können in die üblichen Formulierungen übergeführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z.B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage: z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden und/oder mit Stoffen, welche die Kulturpflanzen-Verträglichkeit verbessern ("Safenern") zur Unkrautbekämpfung verwendet werden, wobei Fertigformulierungen oder Tankmischungen möglich sind. Es sind also auch Mischungen mit Unkrautbekämpfungsmitteln möglich, welche ein oder mehrere bekannte Herbizide und einen Safener enthalten.

Für die Mischungen kommen bekannte Herbizide infrage, beispielsweise

Acetochlor, Acifluorfen (-sodium), Aclonifen, Alachlor, Alloxydim (-sodium), Ametryne, Amicarbazone, Amidochlor, Amidosulfuron, Anilofos, Asulam, Atrazine, Azafenidin, Azimsulfuron, BAS-662H, Beflubutamid, Benazolin (-ethyl), Benfuresate, Bensulfuron (-methyl), Bentazon, Benzfendizone, Benzobicyclon, Benzofenap, Benzoylprop (-ethyl), Bialaphos, Bifenox, Bispyribac (-sodium), Bromobutide, Bromofenoxim, Bromoxynil, Butachlor, Butafenacil (-allyl), Butroxydim, Butylate, Cafenstrole, Caloxydim, Carbetamide, Carfentrazone (-ethyl), Chlomethoxyfen, Chloramben, Chloridazon, Chlorimuron (-ethyl), Chlornitrofen, Chlorsulfuron, Chlortoluron, Cinidon (-ethyl), Cinmethylin, Cinosulfuron, Clefoxydim, Clethodim, Clodinafop (-propargyl), Clomazone, Clomeprop, Clopyralid, Clopyrasulfuron (-methyl), Cloransulam (-methyl), Cumyluron, Cyanazine, Cybutryne, Cycloate, Cyclosulfamuron, Cycloxydim, Cyhalofop (-butyl), 2,4-D, 2,4-DB, Desmedipham, Diallate, Dicamba, Dichlorprop (-P), Diclofop (-methyl), Diclosulam, Diethatyl (-ethyl), Difenzoquat, Diflufenican, Diflufenzopyr, Dimefuron, Dimepiperate, Dimethachlor, Dimethametryn, Dimethenamid, Dimexyflam, Dinitramine, Diphenamid, Diquat, Dithiopyr, Diuron, Dymron, Epropodan, EPTC, Esprocarb, Ethalfluralin, Ethametsulfuron (-methyl), Ethofumesate, Ethoxyfen, Ethoxysulfuron, Etobenzanid, Fenoxaprop (-P-ethyl), Fentrazamide, Flamprop (-isopropyl, -isopropyl-L, -methyl), Flazasulfuron, Florasulam, Fluazifop (-P-butyl), Fluazolate, Flucarbazone (-sodium), Flufenacet, Flumetsulam, Flumiclorac (-pentyl), Flumioxazin, Flumipropyn, Flumetsulam, Fluometuron, Fluorochloridone, Fluoroglycofen (-ethyl), Flupoxam, Flupropacil, Flurpyrsulfuron (-methyl, -sodium), Flurenol (-butyl), Fluridone, Fluroxypyr (-butoxypropyl, -meptyl), Flurprimidol, Flurtamone, Fluthiacet (-methyl), Fluthiamide, Fomesafen, Foramsulfuron, Glufosinate (ammonium), Glyphosate (-isopropylammonium), Halosafen, Haloxyfop (-ethoxyethyl, -P-methyl), Hexazinone, Imazamethabenz (-methyl), Imazamethapyr, Imazamox, Imazapic, Imazapyr, Imazaquin, Imazethapyr, Imazosulfuron, Iodosulfuron (methyl, -sodium), Ioxynil, Isopropalin, Isoproturon, Isouron, Isoxaben, Isoxachlortole, Isoxaflutole, Isoxapyrifop, Lactofen, Lenacil, Linuron, MCPA, Mecoprop, Mefenacet, Mesotrione, Metamitron, Metazachlor, Methabenzthiazuron, Metobenzuron, Metobromuron, (alpha-) Metolachlor, Metosulam, Metoxuron, Metribuzin, Metsulfuron (-methyl), Molinate, Monolinuron, Naproanilide, Napropamide, Neburon, Nicosulfuron, Norflurazon, Orbencarb, Oryzalin, Oxadiargyl, Oxadiazon, Oxasulfuron, Oxaziclomefone, Oxyfluorfen, Paraquat, Pelargonsäure, Pendimethalin, Pendralin, Pentoxazone, Phenmedipham, Picolinafen, Piperophos, Pretilachlor, Primisulfuron (-methyl), Profluazol, Prometryn, Propachlor, Propanil, Propaquizafop, Propisochlor, Procarbazone (-sodium), Propyzamide, Prosulfocarb, Prosulfuron, Pyraflufen (-ethyl), Pyrazogyl, Pyrazolate, Pyrazosulfuron (-ethyl), Pyrazoxyfen, Pyribenzoxim, Pyributicarb, Pyridate, Pyridatol, Pyriftalid, Pyriminobac (-methyl), Pyrithiobac (-sodium), Quinchlorac, Quinmerac, Quinoclamine, Quizalofop (-P-ethyl, -P-tefuryl), Rimsulfuron, Sethoxydim, Simazine, Simetryn, Sulcotrione, Sulfentrazone, Sulfometuron (-methyl), Sulfosate, Sulfosulfuron, Tebutam, Tebuthiuron, Tepraloxydim, Terbuthylazine, Terbutryn, Thenylchlor, Thiafluamide, Thiazopyr, Thidiazimin, Thifensulfuron (-methyl), Thiobencarb, Tiocarbazil, Tralkoxydim, Triallate, Triasulfuron, Tribenuron (-methyl), Triclopyr, Tridiphane, Trifluralin, Trifloxysulfuron, Triflusulfuron (-methyl), Tritosulfuron.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden. Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 1 g und 10 kg Wirkstoff pro Hektar Bodenfläche, vorzugsweise zwischen 5 g und 5 kg pro ha.

Die Herstellung und die Verwendung der erfindungsgemäßen Wirkstoffe geht aus den nachfolgenden Beispielen hervor.

### Herstellungsbeispiele

### Beispiel 1

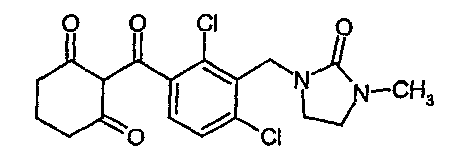

4,4 g (14,5 mMol) 2,4-Dichlor-3-[(3-methyl-2-oxo-imidazolidin-1-yl)-methyl]-benzoesäure werden in 130 ml Acetonitril vorgelegt und bei Raumtemperatur (ca. 20°C) unter Rühren nach einander mit 2,0 g (17,4 mMol) 1,3-Cyclohexandion und 3,6 g (17,4 mMol) Dicyclohexylcarbodiimid versetzt. Die Reaktionsmischung wird 30 Minuten gerührt und dann mit 4,4 g (4,35 mMol) Triethylamin und 2,6 g (29 mMol) Acetoncyanhydrin versetzt. Nach 15 Stunden Rühren wird filtriert, das Filtrat im Wasserstrahlvakuum eingeengt, der Rückstand mit Methylenchlorid / 1N-Salzsäure geschüttelt, die organische Phase abgetrennt, mit 100 ml Wasser versetzt, mit Kaliumcarbonat auf pH 11 eingestellt, die wässrige Phase mit Methylenchlorid geschüttelt, abgetrennt, mit 100 ml Methylenchlorid versetzt und mit 1N-Salzsäure angesäuert. Die organische Phase wird mit Wasser und dann mit gesättigter wässriger Natriumchlorid-Lösung gewaschen, mit Natriumsulfat getrocknet und filtriert. Vom Filtrat wird das Lösungsmittel unter vermindertem Druck sorgfältig abdestilliert.

Man erhält 2,4 g (42% der Theorie) 2-[2,4-Dichlor-3-[(3-methyl-2-oxo-imidazolidin-1-yl)-methyl]-benzoyl]-1,3-cyclohexandion als amorphes Produkt.

Analog zu Beispiel 1 sowie entsprechend der allgemeinen Beschreibung des erfindungsgemäßen Herstellungsverfahrens können beispielsweise auch die in der nachstehenden Tabelle 1 aufgeführten Verbindungen der allgemeinen Formel (I) - bzw. der Formeln (IA), (IB) oder (IC) - hergestellt werden.

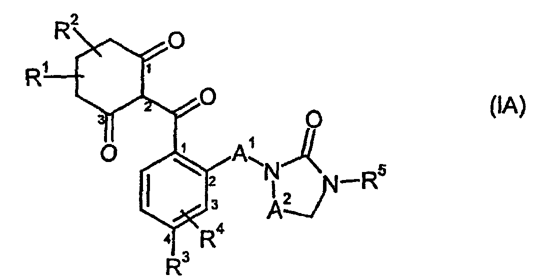

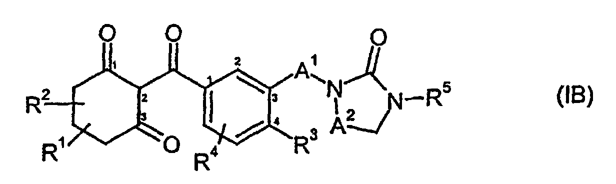

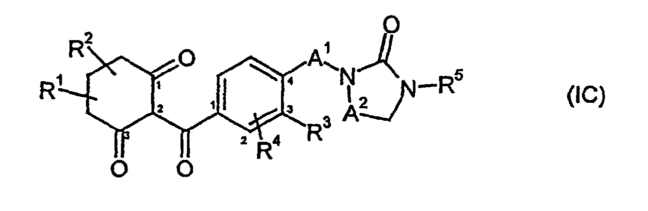

**Tabelle 1:**

| Beispiele für die Verbindungen der Formel (I) | | | | | | | | |
|---|---|---|---|---|---|---|---|---|
| Bsp.-Nr. | A¹ | A² | (Position) R¹ (bzw. R¹ + R²) | (Position) R² | R³ | (Position) R⁴ | R⁵ | Formel Physikal. Daten |
| 2 | CH₂ | CH₂CH₂ | (5) CH₃ | H | Cl | (2) Cl | CH₃ | (IB) logP = 2,25 ^{a)} |
| 3 | CH₂ | CH₂CH₂ | (5) CH₃ | (5) CH₃ | Cl | (2) Cl | CH₃ | (IB) logP = 2,79 ^{a)} |
| 4 | CH₂ | CH₂CH₂ | (4) CH₃ | (4) CH₃ | Cl | (2) Cl | CH₃ | (IB) logP = 2,89 ^{a)} |
| 5 | CH₂ | CH₂CH₂ | (5) C₃H₇-i | H | Cl | (2) Cl | CH₃ | (IB) logP = 3,22 ^{a)} |
| 6 | CH₂ | CH₂CH₂ | (5) C₆H₅ | H | Cl | (2) Cl | CH₃ | (IB) logP = 3,21 ^{a)} |
| 7 | CH₂ | CH₂ | (5) CH₃ | H | Cl | (2) Cl | CH₃ | (IB) logP = 2,43 ^{a)} |
| 8 | CH₂ | CH₂ | (5) CH₃ | (5) CH₃ | Cl | (2) Cl | CH₃ | (IB) logP = 2,67 ^{a)} |
| 9 | CH₂ | CH₂ | (4) CH₃ | (4) CH₃ | Cl | (2) Cl | CH₃ | (IB) logP = 2,75 ^{a)} |
| 10 | CH₂ | CH₂ | (5) C₃H₇-i | H | Cl | (2) Cl | CH₃ | (IB) logP = 3,09 ^{a)} |
| 11 | CH₂ | CH₂ | (5) C₆H₅ | H | Cl | (2) Cl | CH₃ | (IB) logP = 3,10 ^{a)} |
| 12 | CH₂ | CH₂ | (5) CH₃ | H | CI | (2) Cl | C₂H₅ | (IB) logP = 2,71 ^{a)} |
| 13 | CH₂ | CH₂ | H | H | Cl | (2) Cl | C₂H₅ | (IB) logP = 2,37 ^{a)} |
| 14 | CH₂ | CH₂ | (5) CH₃ | (5) CH₃ | Cl | (2) Cl | C₂H₅ | (IB) logP = 2,96 ^{a)} |
| 15 | CH₂ | CH₂ | (4) CH₃ | (4) CH₃ | Cl | (2) Cl | C₂H₅ | (IB) logP = 3,07 ^{a)} |
| 16 | CH₂ | CH₂ | (5) C₃H₇-i | H | Cl | (2) Cl | C₂H₅ | (IB) logP = 3,40 ^{a)} |
| 17 | CH₂ | CH₂ | (5) C₆H₅ | H | Cl | (2) Cl | C₂H₅ | (IB) logP = 3,38 ^{a)} |
| 18 | CH₂ | CH₂CH₂ | H | H | Cl | (2) OCH₃ | CH₃ | (IB) logP = 2,05 ^{a)} |
| 19 | CH₂ | CH₂ | H | H | Cl | (2) OCH₃ | CH₃ | (IB) logP = 2,00 ^{a)} |
| 20 | CH₂ | CH₂ | (5,5) (CH₂)₃ | - | Cl | (2) Cl | CH₃ | (IB) logP = 2,83 ^{a)} |
| 21 | CH₂ | CH₂ | (5,5) (CH₂)₃ | - | Cl | (2) Cl | C₂H₅ | (IB) logP = 3,15 ^{a)} |
| 22 | CH₂ | CH₂ | H | H | SO₂CH₃ | (2) Cl | CH₃ | (IB) logP = 1,70 ^{a)} |
| 23 | CH₂ | CH₂ | H | H | SO₂CH₃ | (2) Cl | C₂H₅ | (IB) logP = 1,91 ^{a)} |
| 24 | CH₂ | CH₂ | H | H | F | (2) Cl | CH₃ | (IB) logP = 1,96 ^{a)} |
| 25 | CH₂ | CH₂ | H | H | CF₃ | - | C₂H₅ | (IA) logP = 2,68 ^{a)} |
| 26 | CH₂ | CH₂ | (5) CH₃ | H | SO₂CH₃ | (2) Cl | CH₃ | (IB) Schmp. 147 °C |
| 27 | CH₂ | CH₂ | (5) CH₃ | (5) CH₃ | SO₂CH₃ | (2) Cl | CH₃ | (IB) Schmp. 142 °C |
| 28 | CH₂ | CH₂ | (4) CH₃ | (4) CH₃ | SO₂CH₃ | (2) Cl | CH₃ | (IB) Schmp. 140 °C |
| 29 | CH₂ | CH₂ | (5) C₃H₇-i | H | SO₂CH₃ | (2) Cl | CH₃ | (IB) Schmp. 162 °C |
| 30 | CH₂ | CH₂ | (5) C₆H₅ | H | SO₂CH₃ | (2) Cl | CH₃ | (IB) Schmp. 174 °C |
| 31 | CH₂ | CH₂ | (5,5) (CH₂)₃ | - | SO₂CH₃ | (2) Cl | CH₃ | (IB) Schmp. 181 °C |
| 32 | CH₂ | CH₂ | (5) CH₃ | H | SO₂CH₃ | (2) Cl | C₂H₅ | (IB) Schmp. 40 °C |
| 33 | CH₂ | CH₂ | (5) CH₃ | (5) CH₃ | SO₂CH₃ | (2) Cl | C₂H₅ | (IB) Schmp. 160 °C |
| 34 | CH₂ | CH₂ | (4) CH₃ | (4) CH₃ | SO₂CH₃ | (2) Cl | C₂H₅ | (IB) Schmp. 166 °C |
| 35 | CH₂ | CH₂ | (5) C₃H₇-i | H | SO₂CH₃ | (2) Cl | C₂H₅ | (IB) Schmp. 144 °C |
| 36 | CH₂ | CH₂ | (5) C₆H₅ | H | SO₂CH₃ | (2) Cl | C₂H₅ | (IB) Schmp. 182 °C |
| 37 | CH₂ | CH₂ | (5,5) (CH₂)₃ (CH₂)₃ | - | SO₂CH₃ | (2) Cl | C₂H₅ | (IB) Schmp. 147 °C |
| 38 | CH₂ | CH₂CH₂ | H | H | Cl | (2) OCH₃ | C₃H₇-i | (IB) |
| 39 | CH2 | CH2 | H | H | Cl | (2) Cl | C₃H₇-i | (IB) Fp.: 109°C |
| 40 | CH₂ | CH₂ | H | H | SO₂CH₃ | (2) Cl | C₃H₇-i | (IB) Fp.: 135°C |
| 41 | CH₂ | CH₂CH₂ | H | H | SO₂CH₃ | (2) Cl | CH₃ | (IB) logP = 1,73 ^{a)} |
| 42 | CH₂ | CH₂ | (5) CH₃ | H | Cl | (2) Cl | C₃H₇-i | (IB) Fp.: 123°C |
| 43 | CH₂ | CH₂ | (5) CH₃ | (5) CH₃ | Cl | (2) Cl | C₃H₇-i | (IB) Fp.: 127°C |
| 44 | CH₂ | CH₂CH₂ | H | H | Cl | (2) Cl | C₃H₇-i | (IB) Fp.: 151°C |
| 45 | CH₂ | CH₂CH₂ | H | H | SO₂CH₃ | (2) Cl | C₃H₇-i | (IB) Fp.: 180°C |
| 46 | CH₂ | CH₂ | H | H | Cl | (2) Cl | C₃H₇-n | (IB) logP = 2,69 ^{a)} |
| 47 | CH₂ | CH₂ | H | H | Cl | (2) Cl | C₄H₉-n | (IB) logP = 3,08 ^{a)} |
| 48 | CH₂ | CH₂ | H | H | Cl | (2) Cl | C₄H₉-i | (IB) logP = 3,03 ^{a)} |
| 49 | CH₂ | CH₂CH₂ | H | H | CF₃ | - | CH₃ | (IA) logP = 2,59 ^{a)} |
| 50 | CH₂ | CH₂ | H | H | F | (2) Cl | C₂H₅ | (IB) logP = 2,18 ^{a)} |
| 51 | CH₂ | CH₂ | H | H | CF₃ | - | CH₃ | (IA) logP = 2,41 ^{a)} |

Die Bestimmung der in Tabelle 1 angegebenen logP-Werte erfolgte gemäß EEC-Directive 79/831 Annex V.A8 durch HPLC (High Performance Liquid Chromatography) an einer Phasenumkehrsäule (C 18). Temperatur: 43°C.
(a) Eluenten für die Bestimmung im sauren Bereich: 0,1% wässrige Phosphorsäure, Acetonitril; linearer Gradient von 10% Acetonitril bis 90% Acetonitril - entsprechende Messergebnisse sind in Tabelle 1 mit ^{a)} markiert.
(b) Eluenten für die Bestimmung im neutralen Bereich: 0,01-molare wässrige Phosphatpuffer-Lösung, Acetonitril; linearer Gradient von 10% Acetonitril bis 90% Acetonitril - entsprechende Messergebnisse sind in Tabelle 1 mit ^{b)} markiert.

Die Eichung erfolgte mit unverzweigten Alkan-2-onen (mit 3 bis 16 Kohlenstoff-atomen), deren logP-Werte bekannt sind (Bestimmung der logP-Werte anhand der Retentionszeiten durch lineare Interpolation zwischen zwei aufeinanderfolgenden Alkanonen).

Die lambda-max-Werte wurden an Hand der UV-Spektren von 200 um bis 400 nm in den Maxima der chromatographischen Signale ermittelt.

### Ausgangsstoffe der Formel (III)

### Beispiel (III-1)

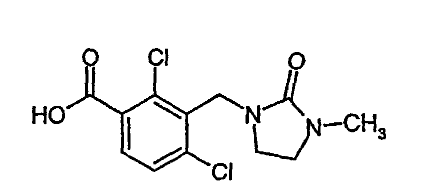

Eine Mischung aus 29 g (88 mMol) 2,4-Dichlor-3-[(3-methyl-2-oxo-imidazolidin-1-yl)-methyl]-benzoesäure-methylester, 3,6 g (90 mMol) Natriumhydroxid, 50 ml Wasser und 100 ml 1,4-Dioxan wird bei Raumtemperatur (ca. 20°C) 15 Stunden gerührt und anschließend im Wasserstrahlvakuum eingeengt. Der Rückstand wird in 100 ml Wasser aufgenommen und unter Rühren mit 2N-Salzsäure angesäuert. Das hierbei kristallin angefallende Produkt wird durch Absaugen isoliert.

Man erhält 25,1 g (94% der Theorie) 2,4-Dichlor-3-[(3-methyl-2-oxo-imidazolidin-1-yl)-methyl]-benzoesäure vom Schmelzpunkt 221°C.

Analog zu Beispiel (III-1) können beispielsweise auch die in der nachstehenden Tabelle 2 aufgeführten Verbindungen der allgemeinen Formel (III) hergestellt werden:

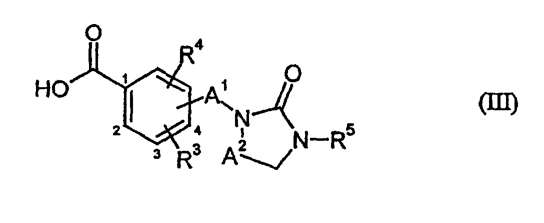

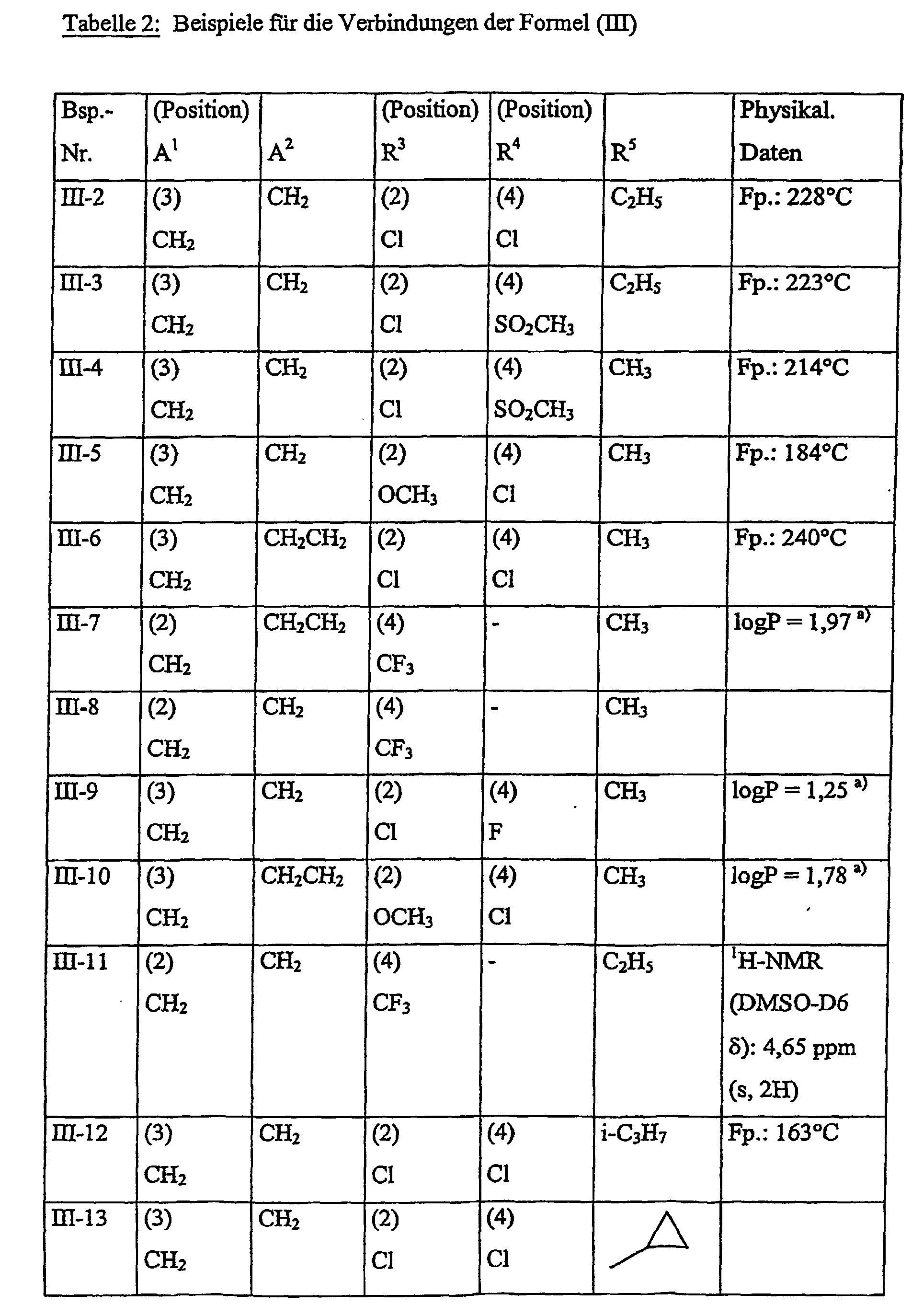

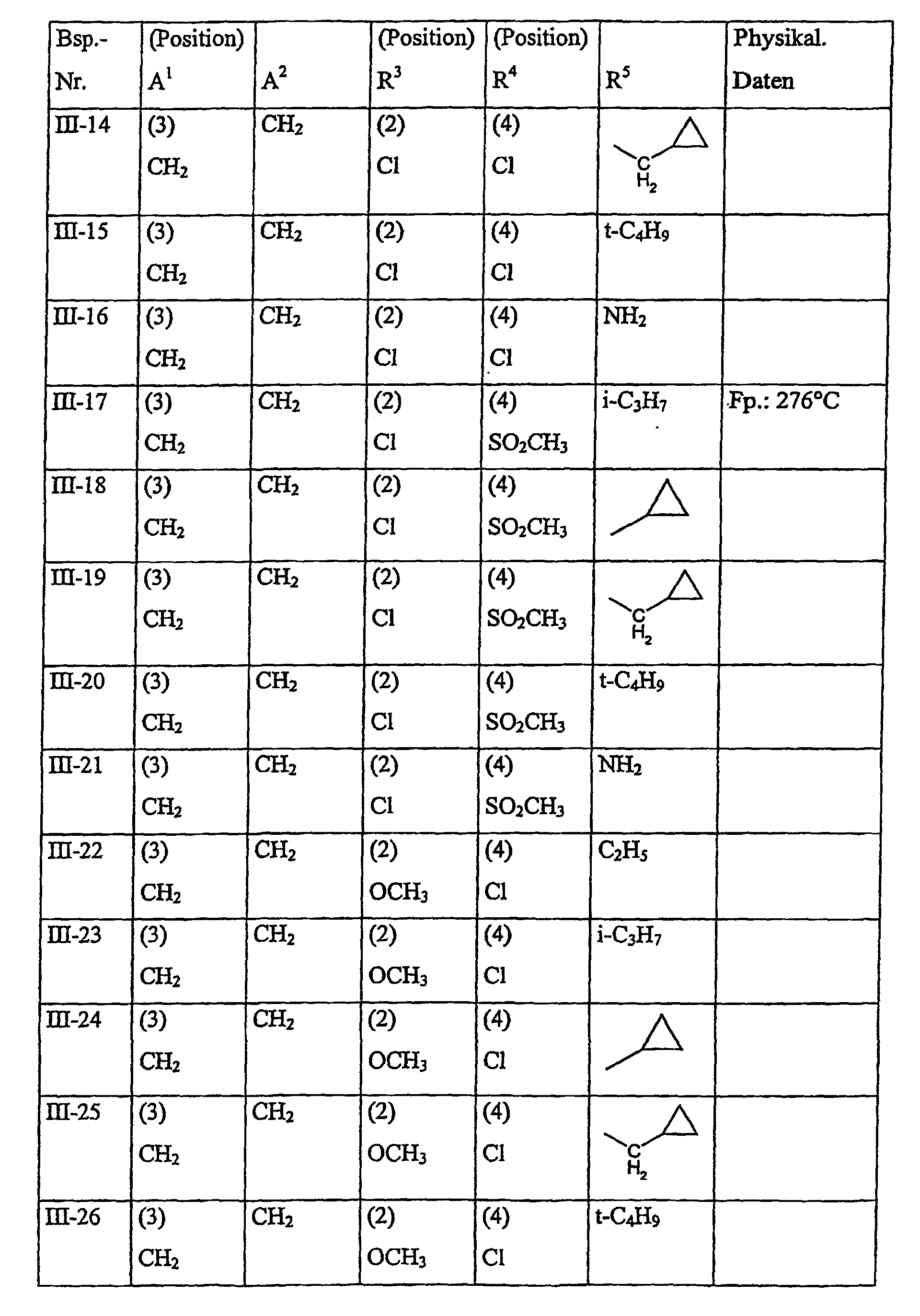

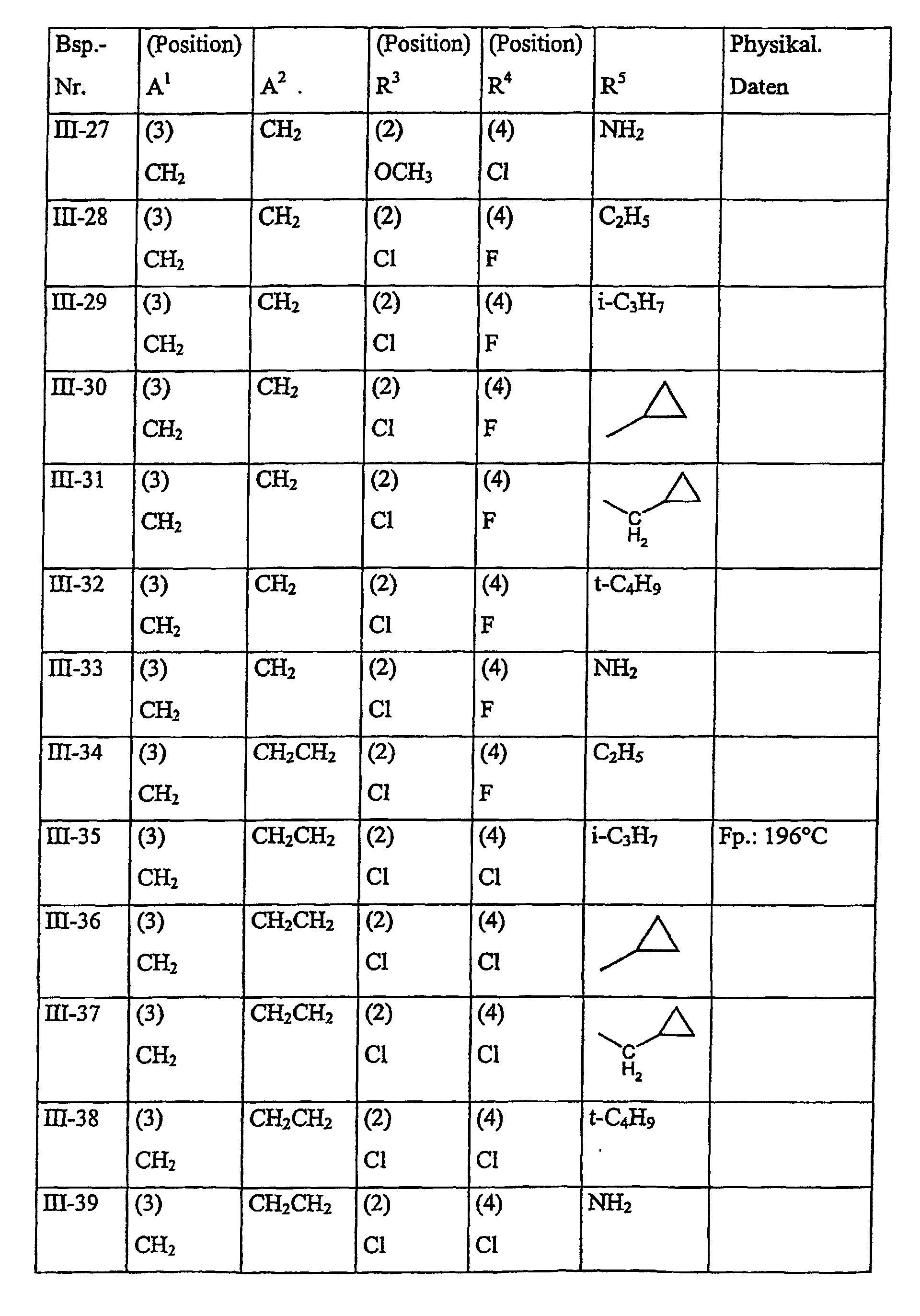

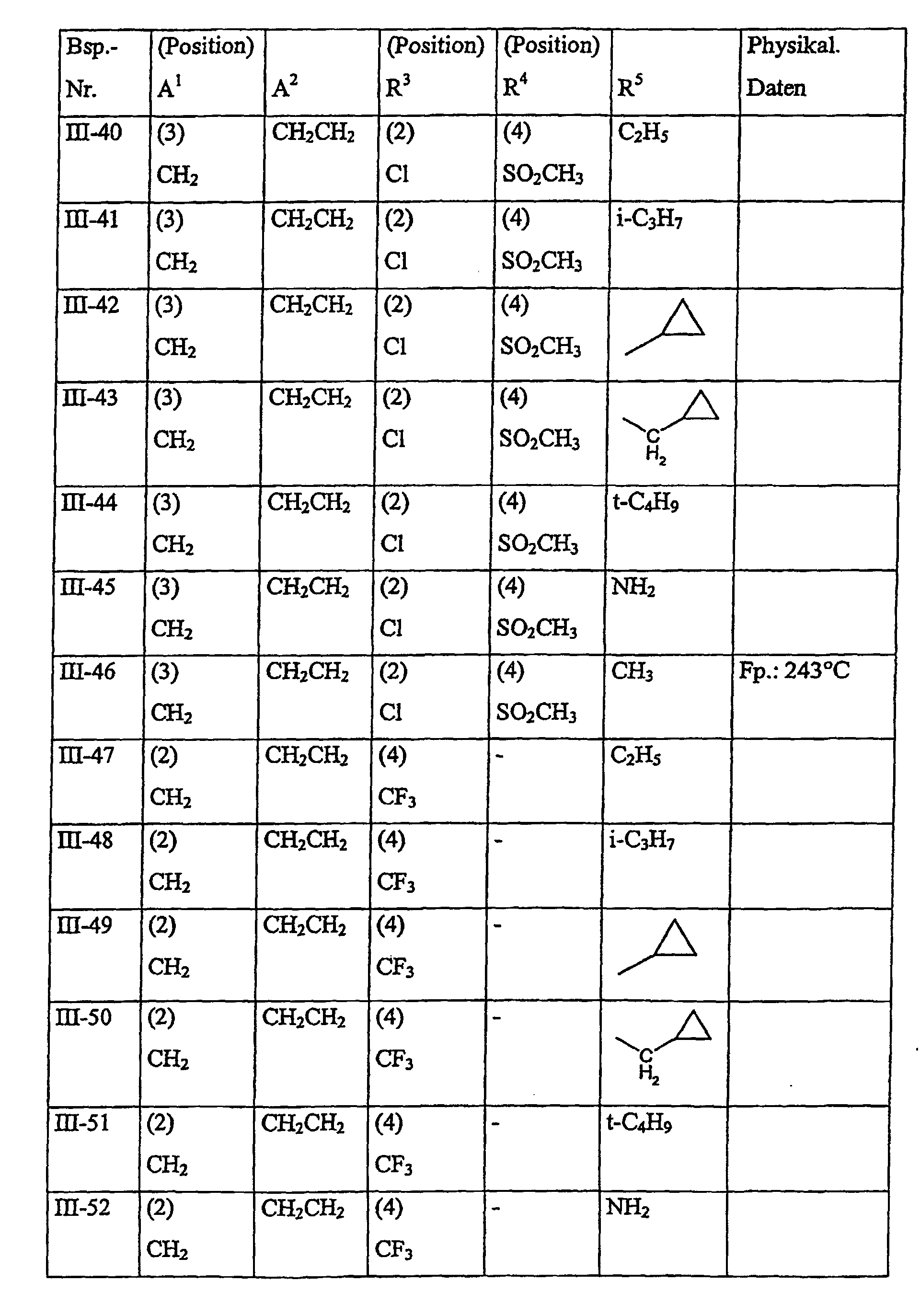

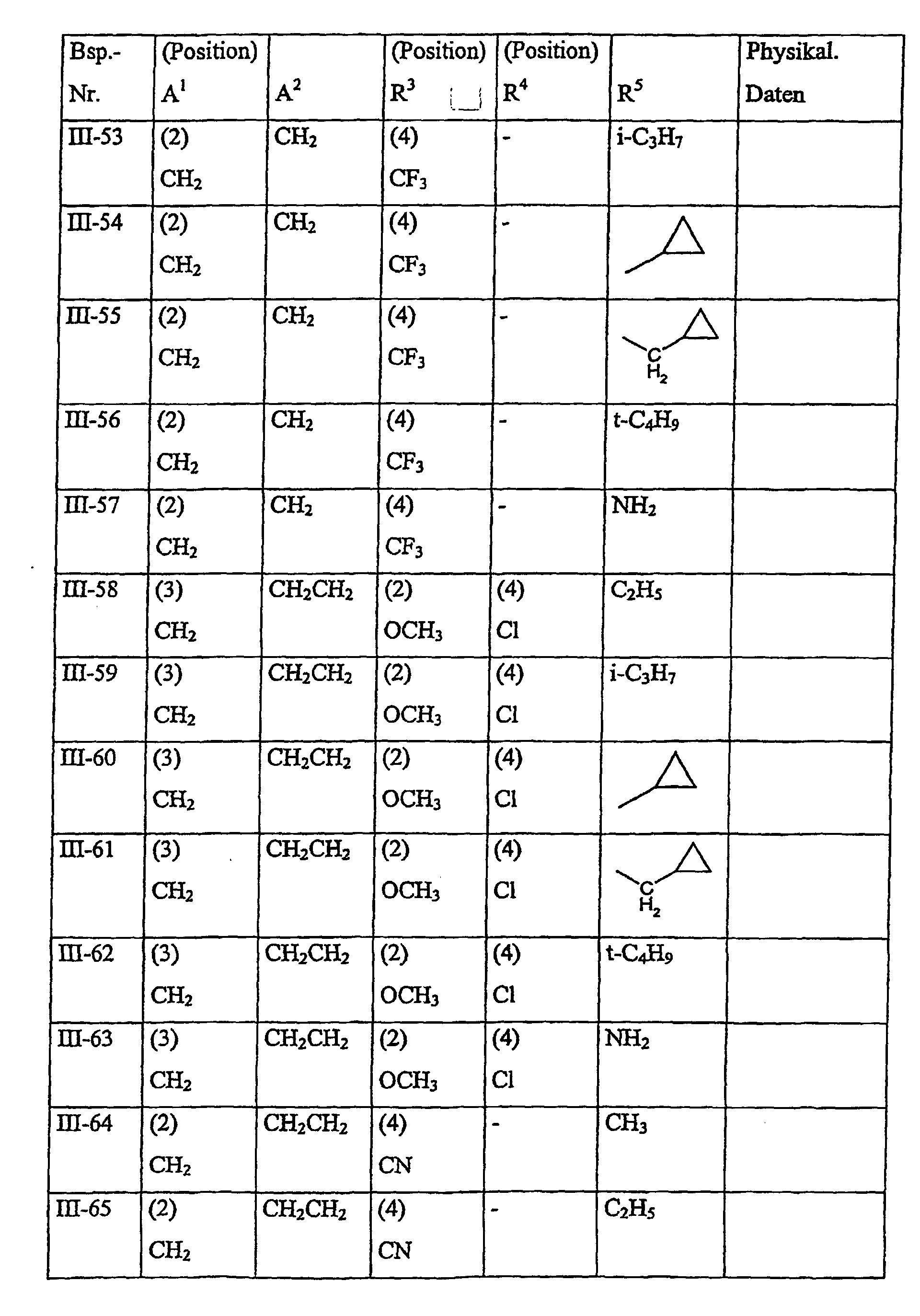

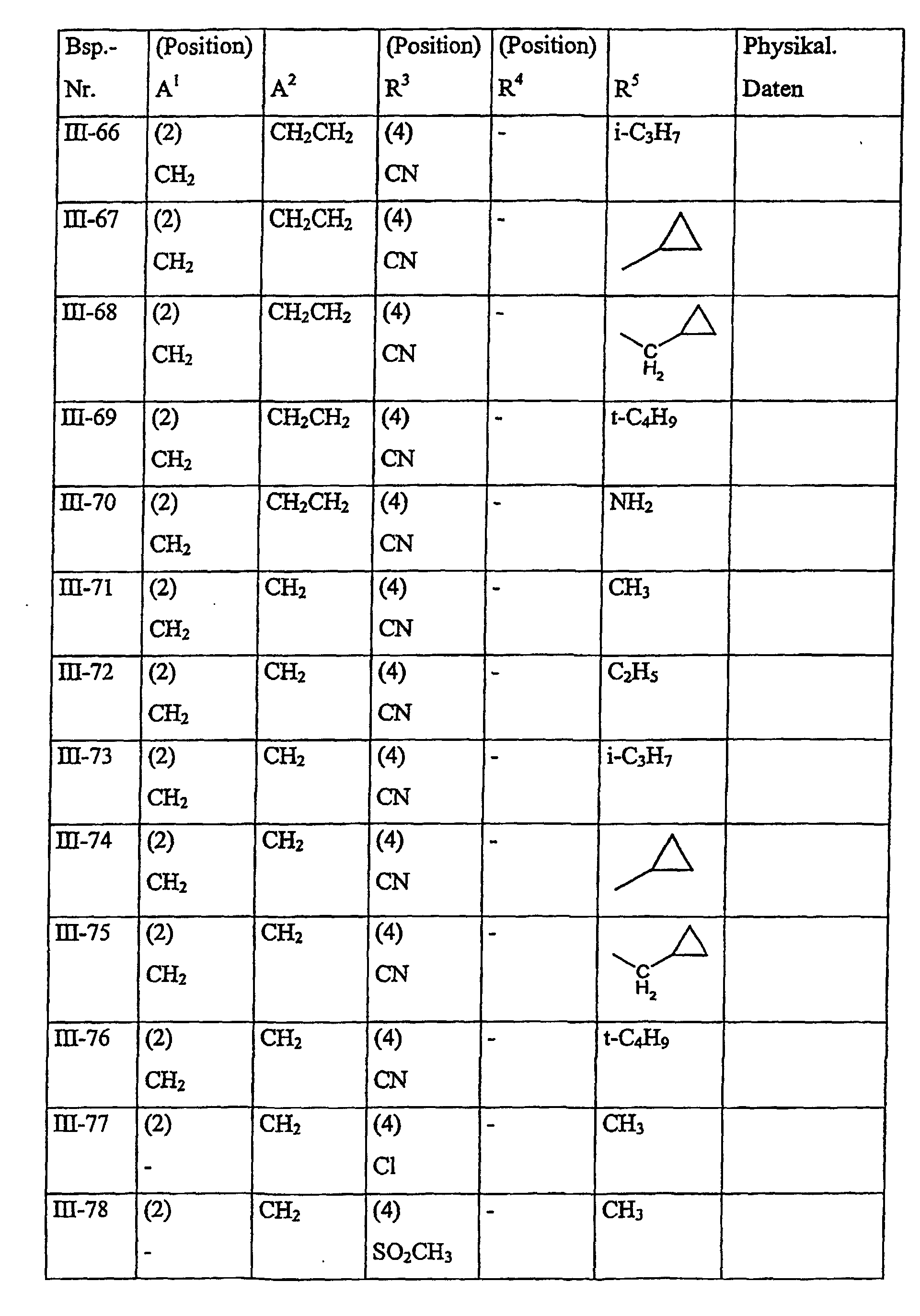

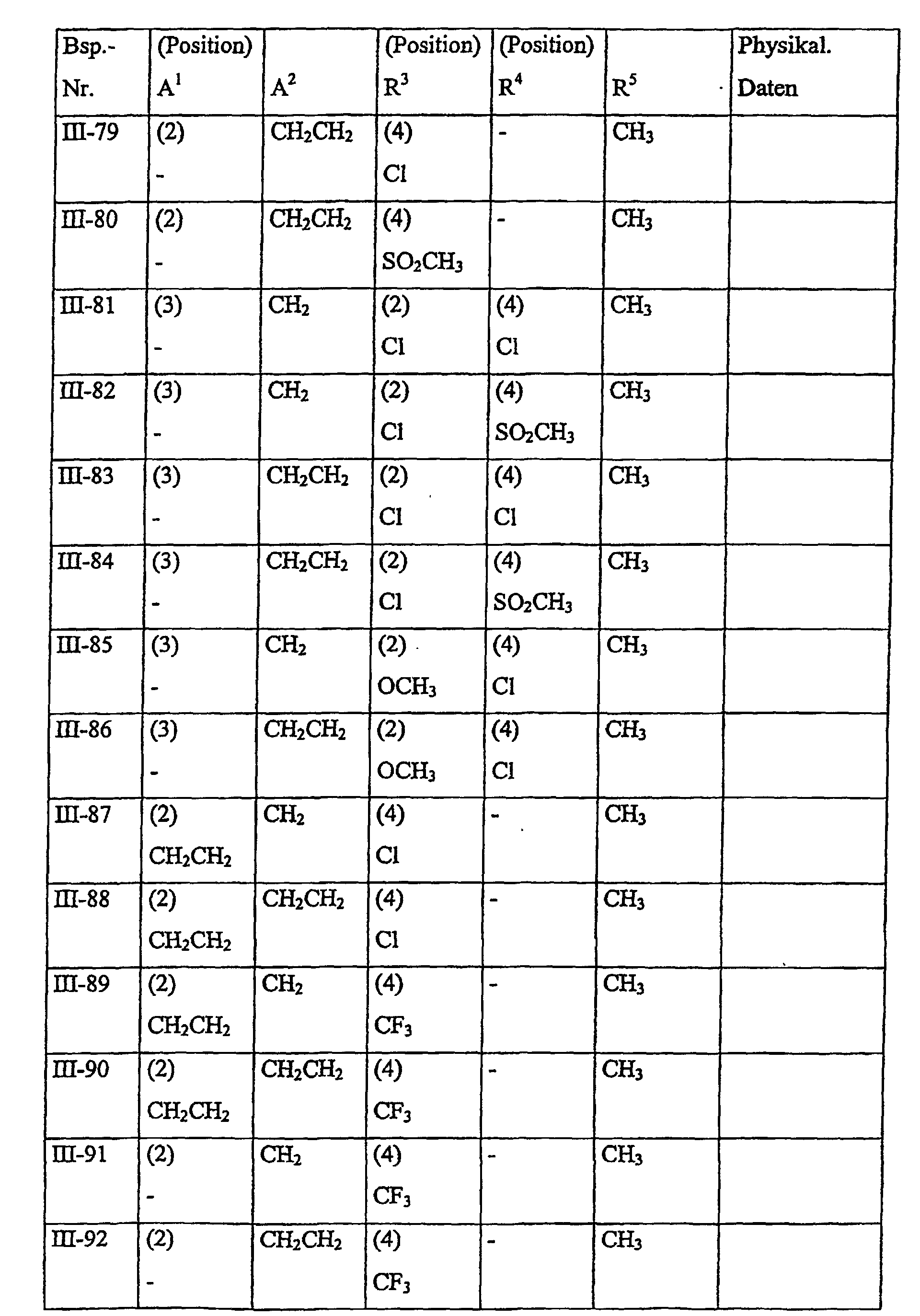

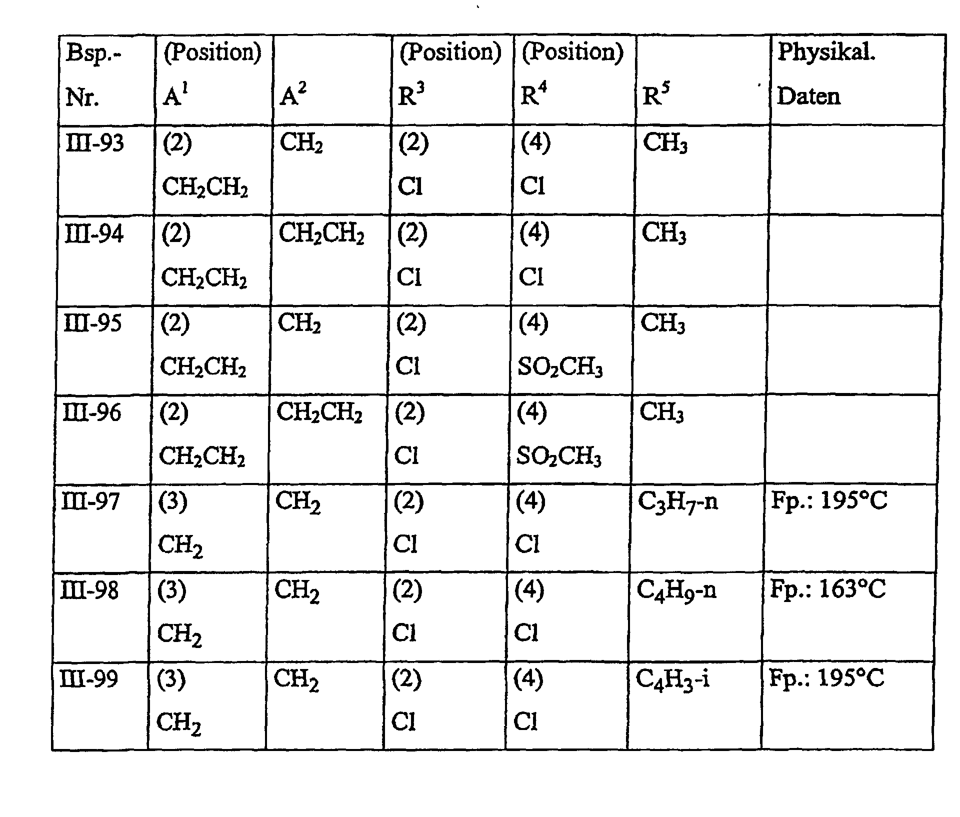

### Vorprodukte der Formel (IV)

### Beispiel (IV-1)

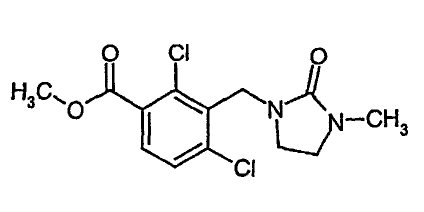

Eine Mischung aus 20 g (0,20 Mol) 1-Methyl-2-oxo-imidazolidin, 30 g (0,10 Mol) 3-Brommethyl-2,4-dichlor-benzoesäure-methylester, 53 g (0,50 Mol) Kaliumcarbonat und 400 ml Acetonitril wird 48 Stunden unter Rühren zum Rückfluss erhitzt. Nach Zugabe von 1 g Natriumiodid wird die Mischung weitere 48 Stunden zum Rückfluss erhitzt und anschließend filtriert. Vom Filtrat wird das Lösungsmittel unter vermindertem Druck sorgfältig abdestilliert. Der Rückstand wird säulenchromatografisch (Kieselgel, Essigsäureethylester) gereinigt.

Man erhält 29,1 g (92% der Theorie) 2,4-Dichlor-3-[(3-methyl-2-oxo-imidazolidin-1-yl)-methyl]-benzoesäure-methylester vom Schmelzpunkt 59°C.

Analog zu Beispiel (IV-1) können beispielsweise auch die in der nachstehenden Tabelle 3 aufgeführten Verbindungen der allgemeinen Formel (IV) - sowie beispielsweise auch die Methylester der oben in Tabelle 2 weiter aufgeführten substituierten Benzoesäuren - hergestellt werden:

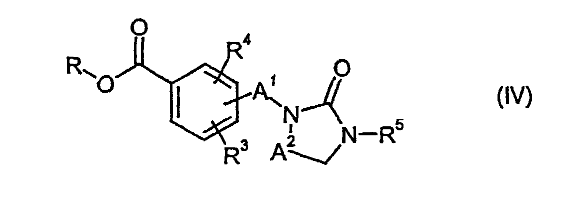

**Tabelle 3:**

| Beispiele für die Verbindungen der Formel (IV) | | | | | | | |
|---|---|---|---|---|---|---|---|
| Bsp.-Nr. | (Position) A¹ | A² | R | (Position) R³ | (Position) R⁴ | R⁵ | Physikal. Daten |
| IV-2 | (3) CH₂ | CH₂ | CH₃ | (2) Cl | (4) Cl | C₂H₅ | logP = 2,26 ^{a)} |
| IV-3 | (3) CH₂ | CH₂ | CH₃ | (2) Cl | (4) SO₂CH₃ | C₂H₅ | logP = 1,55 ^{a)} |
| IV-4 | (3) CH₂ | CH₂ | CH₃ | (2) Cl | (4) SO₂CH₃ | CH₃ | logP *=* 1,55 ^{a)} |
| IV-5 | (3) CH₂ | CH₂ | CH₃ | (2) OCH₃ | (4) Cl | CH₃ | logP = 1,92 ^{a)} |
| IV-6 | (3) CH₂ | CH₂CH₂ | CH₃ | (2) Cl | (4) Cl | CH₃ | logP = 2,07 ^{a)} |
| IV-7 | (2) CH₂ | CH₂CH₂ | CH₃ | (4) CF₃ | - | CH₃ | |
| IV-8 | (2) CH₂ | CH₂ | CH₃ | (4) CF₃ | - | CH₃ | |
| IV-9 | (3) CH₂ | CH₂CH₂ | CH₃ | (2) Cl | (4) F | CH₃ | |
| IV-10 | (3) CH₂ | CH₂CH₂ | CH₃ | (2) OCH₃ | (4) Cl | CH₃ | logP = 2,03 ^{a)} |
| IV-11 | (2) CH₂ | CH₂ | CH₃ | (4) CF₃ | - | C₂H₅ | logP = 2,62 ^{a)} |
| IV-12 | (3) CH₂ | CH₂ | CH₃ | (2) Cl | (4) Cl | C₃H₇-n | logP = 2,62 |
| IV-13 | (3) CH₂ | CH₂ | CH₃ | (2) Cl | (4) Cl | C₃H₇-i | logP = 2,56 |
| IV-14 | (3) CH₂ | CH₂ | CH₃ | (2) Cl | (4) SO₂CH₃ | C₃H₇-i | ¹H-NMR (CD₃CN) δ = 1,07 (d,6H); 3,20 (s,SO₂CH₃); 3,92 (s,OCH₃); 4,91 (CH₂); 7,76 (d,2H); 8,10 (d,2H) ppm |
| IV-15 | (3) CH₂ | CH₂ | CH₃ | (2) Cl | (4) Cl | C₄H₉-n | logP = 3,0 |
| IV-16 | (3) CH₂ | CH₂ | CH₃ | (2) Cl | (4) Cl | C₄H₉-i | logP = 2,95 |
| IV-17 | (3) CH₂ | CH-CH₂ | CH₃ | (2) Cl | (4) SO₂CH₃ | CH₃ | LC-MS: M+1 = 375/7 |
| IV-18 | (3) CH₂ | CH-CH₂ | CH₃ | (2) Cl | (4) Cl | C₃H₇-i | logP = 2,65 |

### Vorprodukte der Formel (V)

Die Halogenalkylbenzoesäureester der allgemeinen Formel (V) können auf bekannte Weise durch Halogenierung von entsprechenden Alkylbenzoesäureester, beispielsweise mit N-Brom-succinimid oder N-Chlor-succinimid, hergestellt werden.

Beispiele für die Halogenalkylbenzoesäureester der Formel (V) sind in der nachstehenden Tabelle 4 aufgeführt.

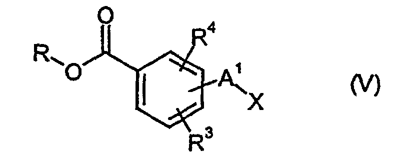

**Tabelle 4:**

| Beispiele für die Verbindungen der Formel (V) | | | | | |
|---|---|---|---|---|---|
| Bsp.-Nr. | (Position) A¹X | (Position) R³ | (Position) R⁴ | R | Physikal. Daten |
| V-1 | (3) CH₂Br | (2) Cl | (4) Cl | CH₃ | Fp.:61°C |
| V-2 | (3) CH₂Br | (2) Cl | (4) SO₂CH₃ | CH₃ | logP = 2,33 ^{a)} |
| V-3 | (2) CH₂Br | (4) NO₂ | - | C₂H₅ | ¹H-NMR (CDCl₃, δ): 4,97 ppm (s, CH₂) |
| V-4 | (2) CH₂Br | (4) CF₃ | - | CH₃ | |
| V-5 | (2) CH₂Br | (4) SO₂CH₃ | - | C₂H₅ | ¹H-NMR (CDCl₃, δ): 4,96 ppm (s, CH₂) |
| V-6 | (2) CH₂Br | (4) SO₂CH₃ | - | CH₃ | logP = 1,87 ^{a)} |
| V-7 | (2) CH₂Br | (4) CF₃ | | C₂H₅ | ¹H-NMR (CDCl₃, δ): 4,96 ppm (s, CH₂) |
| V-8 | (2) CH₂Br | (4) F | - | C₂H₅ | ¹H-NMR (CDCl₃, δ): 4,93 ppm (s, CH₂) |
| V-9 | (2) CH₂Br | (4) Cl | - | C₂H₅ | ¹H-NMR (CDCl₃, δ): 4,90 ppm (s, CH₂) |
| V-10 | (2) CH₂Br | (4) Br | - | C₂H₅ | ¹H-NMR (CDCl₃, δ): 4,90 ppm (s, CH₂) |
| V-11 | (2) CH₂Br | (4) OCH₃ | - | C₂H₅ | |
| V-12 | (2) CH₂Br | (4) I | - | CH₃ | |
| V-13 | (3) CH₂Br | (2) OCH₃ | (4) Cl | CH₃ | ¹H-NMR (DMSO-D6, δ): 4,72 ppm (s, CH₂) |
| V-14 | (3) CH₂Br | (2) OCH₃ | (4) SO₂CH₃ | CH₃ | |
| V-15 | (3) CH₂Br | (2) OCH₃ | (4) CF₃ | CH₃ | |
| V-16 | (3) CH₂Br | (2) OCH₃ | (4) SCH₃ | CH₃ | |
| V-17 | (3) CH₂Br | (2) NO₂ | (4) CF₃ | CH₃ | |
| V-18 | (3) CH₂Br | (2) NO₂ | (4) SO₂CH₃ | CH₃ | |
| V-19 | (3) CH₂Br | (2) NO₂ | (4) CN | CH₃ | |
| V-20 | (2) CH₂Br | (4) CN | - | CH₃ | |
| V-21 | (3) CH₂Br | (2) CF₃ | (4) SO₂CH₃ | CH₃ | |
| V-22 | (3) CH₂Br | (2) CF₃ | (4) CN | CH₃ | |
| V-23 | (3) CH₂Br | (2) Cl | (4) F | CH₃ | |
| V-24 | (3) CH₂Br | (2) F | (4) Cl | CH₃ | |
| V-25 | (3) CH₂Br | (2) SO₂CH₃ | (4) Cl | CH₃ | |
| V-26 | (4) CH₂Br | (2) Cl | - | CH₃ | |
| V-27 | (2) CH₂Br | (4) Cl | (3) OCH₃ | CH₃ | |

### Vorprodukte der Formel (VI)

### Beispiel (VI-1)

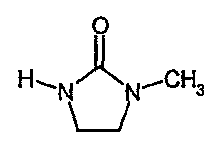

75 g (1,0 Mol) N¹-Methyl-ethan-1,2-diamin werden zu einer Mischung aus 214 g (1,0 Mol) Diphenylcarbonat und 1 Liter Toluol tropfenweise unter Rühren gegeben. Die Mischung wird dann 60 Minuten unter Rückfluss zum Sieden erhitzt und anschließend bei 100°/30 mbar eingeengt. Der Rest wird bei 110°C/1 mbar destilliert und das Destillat (41,5% 1-Methyl-2-oxo-imidazolidin neben 58,5% Phenol) durch Säulenchromatografie (Kieselgel, Essigsäureethylester) gereinigt.

Man erhält 37,3 g (37% der Theorie) 1-Methyl-2-oxo-imidazolidin vom Schmelzpunkt 114°C.

Analog zu Beispiel (VI-1) können beispielsweise auch die in der nachstehenden Tabelle 5 aufgeführten Verbindungen der allgemeinen Formel (VI) hergestellt werden.

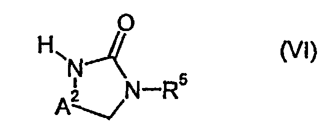

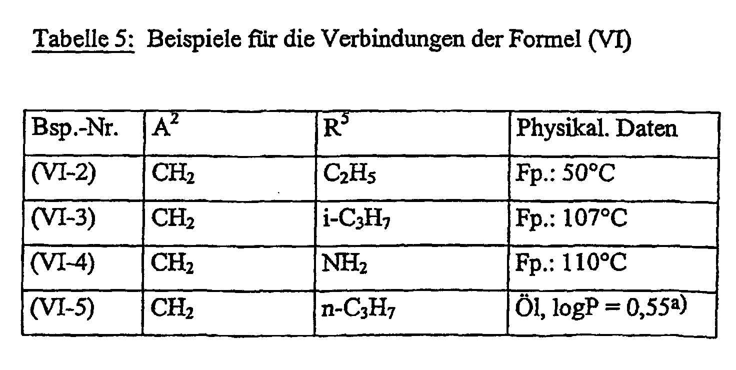

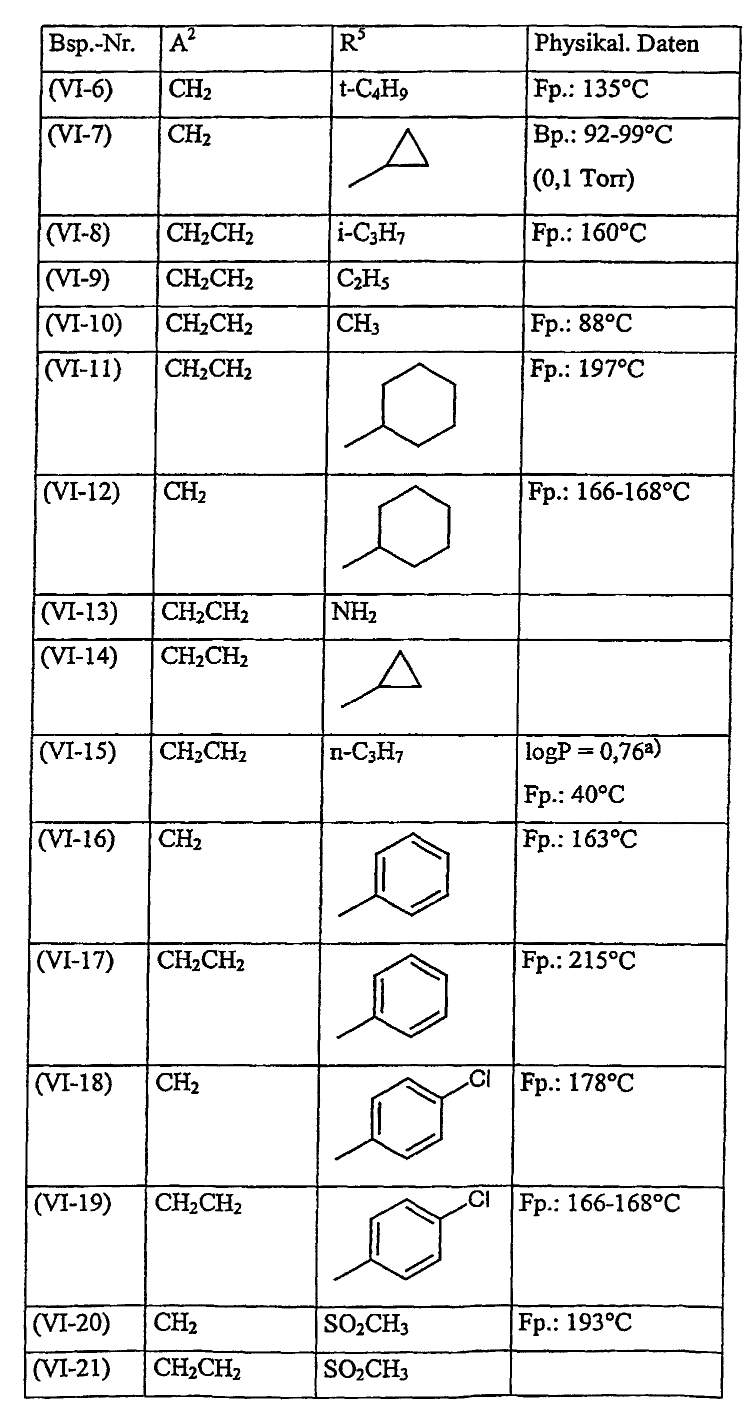

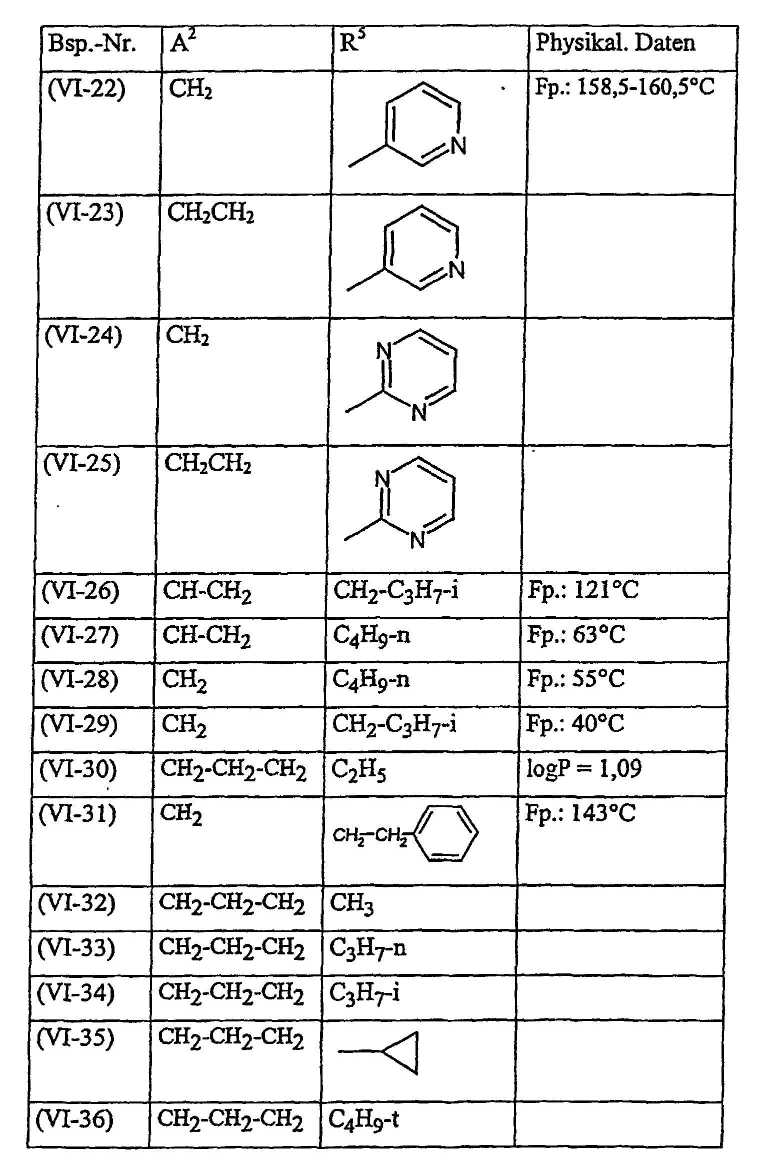

### Anwendungsbeispiele

### Beispiel A

Pre-emergence-Test
- Lösungsmittel:: 5 Gewichtsteile Aceton
- Emulgator:: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät. Nach 24 Stunden wird der Boden so mit der Wirkstoffzubereitung besprüht, dass die jeweils gewünschte Wirkstoffmenge pro Flächeneinheit ausgebracht wird. Die Wirkstoffkonzentration in der Spritzbrühe wird so gewählt, dass in 1000 Liter Wasser pro Hektar die jeweils gewünschte Wirkstoffinenge ausgebracht wird.

Nach drei Wochen wird der Schädigungsgrad der Pflanzen bonitiert in % Schädigung im Vergleich zur Entwicklung der unbehandelten Kontrolle. Es bedeuten:
0 % = keine Wirkung (wie unbehandelte Kontrolle)
100 % = totale Vernichtung

In diesem Test zeigen beispielsweise die Verbindungen gemäß Herstellungsbeispiel 1, 2, 3, 4, 7, 8, 9,12, 13, 18 und 24 starke Wirkung gegen Unkräuter.

### Beispiel B

Post-emergence-Test
- Lösungsmittel:: 5 Gewichtsteile Aceton
- Emulgator:: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben so, dass die jeweils gewünschten Wirkstoffmengen pro Flächeneinheit ausgebracht werden. Die Konzentration der Spritzbrühe wird so gewählt, dass in 1000 1 Wasser/ha die jeweils gewünschten Wirkstoffmengen ausgebracht werden.

Nach drei Wochen wird der Schädigungsgrad der Pflanzen bonitiert in % Schädigung im Vergleich zur Entwicklung der unbehandelten Kontrolle.

Es bedeuten:
0 % = keine Wirkung (wie unbehandelte Kontrolle)
100 % = totale Vernichtung

In diesem Test zeigen beispielsweise die Verbindungen gemäß Herstellungsbeispiel 1, 2, 3, 4, 5, 6, 7, 8, 9, 10, 11, 12, 13, 14, 16, 17, 18 und 24 starke Wirkung gegen Unkräuter.

## Patentansprüche

1. Substituierte Benzoylcyclohexandione der allgemeinen Formel (I), 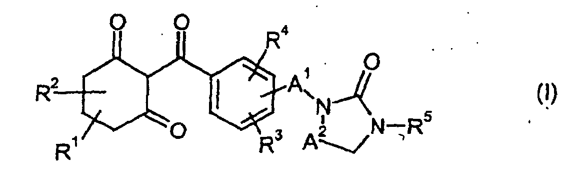 in welcher
A¹ für Alkandiyl (Alkylen) mit 1 bis 3 Kohlenstoffatomen steht,
A² für Alkandiyl (Alkylen) mit 1 bis 3 Kohlenstoffatomen steht,
R¹ für Wasserstoff, für Phenyl oder für jeweils gegebenenfalls durch Halogen substituiertes Alkyl, Alkylthio oder Alkoxycarbonyl mit jeweils 1 bis 4 Kohlenstoffatomen in den Alkylgruppen steht,
R² für Wasserstoff oder für jeweils gegebenenfalls durch Halogen substituiertes Alkyl oder Alkylthio mit jeweils 1 bis 4 Kohlenstoffatomen steht, oder zusammen mit R¹ für Alkandiyl (Alkylen) mit bis zu 4 Kohlenstoffatomen steht, oder - für den Fall, dass R¹ und R² an das gleiche Kohlenstoffatom gebunden sind - zusammen mit R¹ und dem Kohlenstoffatom, an das R¹ und R² gebunden sind, für eine Carbonyl-Gruppierung (C=O) steht,
R³ für Wasserstoff, Nitro, Cyano, Carboxy, Carbamoyl, Thiocarbamoyl, Halogen, für jeweils gegebenenfalls durch Halogen substituiertes Alkyl, Alkoxy, Alkylthio, Alkylsulfinyl, Alkylsulfonyl, Alkylamino, Dialkylamino, Alkylsulfonylamino oder Dialkylaminosulfonyl mit jeweils 1 bis 4 Kohlenstoffatomen in den Alkylgruppen, oder für jeweils gegebenenfalls durch Halogen oder C₁-C₄-Alkyl substituiertes Cycloalkylamino, Cycloalkylsulfonyl, Cycloalkylsulfonylamino oder Cycloalkylaminosulfonyl mit jeweils 3 bis 6 Kohlenstoffatomen steht,
R⁴ für Wasserstoff, Nitro, Cyano, Carboxy, Carbamoyl, Thiocarbamoyl, Halogen, oder für jeweils gegebenenfalls durch Halogen substituiertes Alkyl, Alkoxy, Alkylthio, Alkylsulfinyl, Alkylsulfonyl, Alkylamino, Dialkylamino oder Dialkylaminosulfonyl mit jeweils 1 bis 4 Kohlenstoffatomen in den Alkylgruppen steht, und
R⁵ für Wasserstoff, für Amino, für jeweils gegebenenfalls durch Amino, Cyano, Halogen oder C₁-C₄-Alkoxy substituiertes Alkyl, Alkoxy, Alkylamino, Dialkylamino, Alkylsulfonyl oder Alkylsulfonylamino mit jeweils 1 bis 5 Kohlenstoffatomen in den Alkylgruppen, für jeweils gegebenenfalls durch Halogen substituiertes Alkenyl oder Alkinyl mit jeweils bis zu 5 Kohlenstoffatomen, für jeweils gegebenenfalls durch Cyano, Halogen oder C₁-C₄-Alkyl substituiertes Cycloalkyl oder Cycloalkylalkyl mit jeweils 3 bis 6 Kohlenstoffatomen in der Cycloalkylgruppe und gegebenenfalls 1 bis 3 Kohlenstoffatomen im Alkylteil, für Cycloalkenyl mit 5 oder 6 Kohlenstoffatomen, oder für jeweils gegebenenfalls durch Nitro, Cyano, Carboxy, Halogen, C₁-C₄-Alkyl, C₁-C₄-Halogenalkyl, C₁-C₄-Alkoxy oder C₁-C₄-Halogenalkoxy substituiertes Phenyl, Phenyl-C₁-C₄-alkyl, Pyridinyl oder Pyrimidinyl steht,
einschließlich aller möglichen tautomeren Formen der Verbindungen der allgemeinen Formel (I) und die möglichen Salze bzw. Metallkomplexe der Verbindungen der allgemeinen Formel (I),
ausgenommen die Verbindung 1-[2,6-Dichlor-3-[(2-hydroxy-6-oxo-cyclohexen-1-yl)-carbonyl]-benzyl]-3-methyl-tetrahydro-2(1H)-pyrimidinon.

2. Verbindungen gemäß Anspruch 1, **dadurch gekennzeichnet, dass**
A¹ für Methylen, Ethan-1,1-diyl (Ethyliden), Ethan-1,2-diyl (Dimethylen), Propan-1,1-diyl, Propan-1,2-diyl oder Propan-1,3-diyl steht,
A² für Methylen, Ethan-1,1-diyl (Ethyliden), Ethan-1,2-diyl (Dimethylen), Propan-1,1-diyl, Propan-1,2-diyl oder Propan-1,3-diyl steht,
R¹ für Wasserstoff, für Phenyl oder für jeweils gegebenenfalls durch Fluor und/oder Chlor substituiertes Methyl, Ethyl, n- oder i-Propyl, Methylthio, Ethylthio, n- oder i-Propylthio, Methoxycarbonyl, Ethoxycarbonyl, n- oder i-Propoxycarbonyl steht,
R² für Wasserstoff oder für jeweils gegebenenfalls durch Fluor und/oder Chlor substituiertes Methyl, Ethyl, n- oder i-Propyl, Methylthio, Ethylthio, n- oder i-Propylthio, oder zusammen mit R¹ für Methylen, Ethan-1,1-diyl, Ethan-1,2-diyl (Dimethylen), Propan-1,1-diyl, Propan-1,2-diyl oder Propan-1,3-diyl, oder - für den Fall, dass R¹ und R² an das gleiche Kohlenstoffatom gebunden sind - zusammen mit R¹ und dem Kohlenstoffatom, an das R¹ und R² gebunden sind, für eine Carbonyl-Gruppierung (C=O) steht,
R³ für Wasserstoff, Nitro, Cyano, Carboxy, Carbamoyl, Thiocarbarnoyl, Fluor, Chlor, Brom, lod, für jeweils gegebenenfalls durch Fluor und/oder Chlor substituiertes Methyl, Ethyl, n- oder i-Propyl, Methoxy, Ethoxy, n- oder i-Propoxy, Methylthio, Ethylthio, n- oder i-Propylthio, Methylsulfinyl, Ethylsulfinyl, n- oder i-Propylsulfinyl, Methylsulfonyl, Ethylsulfonyl, n- oder i-Propylsulfonyl, für Methylamino, Ethylamino, n- oder i-Propylamino, Dimethylamino, Diethylamino, Methylsulfonylamino, Ethylsulfonylamino, n- oder i-Propylsulfonylamino, Dimethylaminosulfonyl oder Diethylaminosulfonyl, oder für jeweils gegebenenfalls durch Fluor, Chlor oder Methyl substituiertes Cyclopropylamino, Cyclobutylamino, Cyclopentylamino, Cyclohexylamino, Cyclopropylsulfonyl, Cyclobutylsulfonyl, Cyclopentylsulfonyl, Cyclohexylsulfonyl, Cyclopropylsulfonylamino, Cyclobutylsulfonylamino, Cyclopentylsulfonylamino, Cyclohexylsulfonylamino, Cyclopropylaminosulfonyl, Cyclobutylaminosulfonyl, Cyclopentylaminosulfonyl oder Cyclohexylaminosulfonyl steht,
R⁴ für Wasserstoff, Nitro, Cyano, Carboxy, Carbamoyl, Thiocarbamoyl, Fluor, Chlor, Brom, lod, oder für jeweils gegebenenfalls durch Fluor und/oder Chlor substituiertes Methyl, Ethyl, n- oder i-Propyl, Methoxy, Ethoxy, n- oder i-Propoxy, Methylthio, Ethytthio, n- oder i-Propylthio, Methylsulfinyl, Ethylsulfinyl, n- oder i-Propylsulfinyl, Methylsulfonyl, Ethylsulfonyl, n- oder i-Propylsulfonyl, für Methytamino, Ethylamino, n- oder i-Propylamino, Dimethylamino, Diethylamino, Dimethylaminosulfonyl oder Diethylaminosulfonyl steht, und
R⁵ für Wasserstoff, für Amino, für jeweils gegebenenfalls durch Amino, Cyano, Fluor und/oder Chlor, Methoxy oder Ethoxy substituiertes Methyl, Ethyl, n- oder i-Propyl, n-, i-, s- oder t-Butyl, Methoxy, Ethoxy, n- oder i-Propoxy, n-, i-, s- oder t-Butoxy, Methylamino, Ethylamino, n- oder i-Propylamino, n-, i-, s- oder t-Butylamino, Dimethylamino, Diethylamino, Dipropylamino, methylsulfonyl, Ethylsulfonyl oder Methylsulfonylamino, für jeweils gegebenenfalls durch Fluor, Chlor und/oder Brom substituiertes Propenyl, Butenyl, Propinyl oder Butinyl, für jeweils gegebenenfalls durch Cyano, Fluor, Chlor, Methyl oder Ethyl substituiertes Cyclopropyl, Cyclobutyl, Cyclopentyl, Cyclohexyl, Cyclopropylmethyl, Cyclobutylmethyl, Cyclopentylmethyl oder Cyclohexylmethyl, für Cyclohexenyl, oder für jeweils gegebenenfalls durch Fluor, Chlor, Brom, Methyl, Trifluormethyl, Methoxy, Difluormethoxy oder Trifluormethoxy substituiertes Phenyl, Benzyl, Pyridinyl oder Pyrimidinyl steht.

3. Verbindungen gemäß Anspruch 1, **dadurch gekennzeichnet, dass**
A¹ für Methylen, Ethan-1,2-diyl (Dimethylen) oder Propan-1,3-diyl steht,
A² für Methylen, Ethan-1,2-diyl (Dimethylen) oder Propan-1,3-diyl steht,
R¹ für Wasserstoff, Phenyl, Methyl, Ethyl, n- oder i-Propyl, Methylthio, Ethylthio, Methoxycarbonyl oder Ethoxycarbonyl steht,
R² für Wasserstoff, Methyl, Ethyl, Methylthio, Ethylthio, oder zusammen mit R' für Methylen, Ethan-1,2-diyl (Dimethylen) oder Propan-1,3-diyl (Trimethylen), oder - für den Fall, dass R¹ und R² an das gleiche Kohlenstoffatom gebunden sind - zusammen mit R¹ und dem Kohlenstoffatom, an das R¹ und R² gebunden sind, für eine Carbonyl-Gruppierung (C=O) steht,
R³ für Wasserstoff, Nitro, Cyano, Fluor, Chlor, Brom, lod, für jeweils gegebenenfalls durch Fluor und/oder Chlor substituiertes Methyl, Ethyl, Methoxy, Ethoxy, Methylthio, Ethylthio, Methylsulfinyl, Ethylsulfinyl, Methylsulfonyl, Ethylsulfonyl, für Methylamino, Ethylamino, Dimethylamino oder Dimethylaminosulfonyl steht,
R⁴ für Wasserstoff, Nitro, Cyano, Fluor, Chlor, Brom, lod, für jeweils gegebenenfalls durch Fluor und/oder Chlor substituiertes Methyl, Ethyl, Methoxy, Ethoxy, Methylthio, Ethylthio, Methylsulfinyl, Ethylsulfinyl, Methylsulfonyl, Ethylsulfonyl, für Methylamino, Ethylamino, Dimethylamino oder Dimethylaminosulfonyl steht, und
R⁵ für Wasserstoff, Methyl, Ethyl, n- oder i-Propyl, n-, i-, s- oder t-Butyl, Propenyl, Propinyl, Cyclopropyl, Cyclopentyl, Cyclohexyl, Cyclopropyimethyl, Cyclopentylmethyl, Cyclohexylmethyl, Phenyl oder Benzyl steht.

4. Verbindungen gemäß Anspruch 1, **dadurch gekennzeichnet, dass**
A¹ für Methylen oder Ethan-1,2-diyl (Dimethylen) steht,
A² für Methylen oder Ethan-1,2-diyl (Dimethylen) steht,
R¹ für Wasserstoff, Phenyl, Methyl, Ethyl, n- oder i-Propyl, Methylthio oder Ethylthio steht,
R² für Wasserstoff, Methyl, Ethyl, Methylthio, oder zusammen mit R¹ für Ethan-1,2-diyl oder Propan-1,3-diyl, oder - für den Fall, dass R¹ und R² an das gleiche Kohlenstoffatom gebunden sind - zusammen mit R¹ und dem Kohlenstoffatom, an das R¹ und R² gebunden sind, für eine Carbonyl-Gruppierung (C=O) steht,
R³ für Wasserstoff, Nitro, Cyano, Fluor, Chlor, Brom, Methyl, Difluormethyl, Trifluormethyl, Dichlormethyl, Trichlormethyl, Chlordifluormethyl, Ethyl, Methoxy, Difluormethoxy, Trifluormethoxy, Chlordifluormethoxy, Ethoxy, Methylthio, Difluormethylthio, Trifluormethylthio, Chlordifluormethylthio, Ethylthio, Methylsulfinyl, Trifluormethylsulfinyl, Ethylsulfinyl, Methylsulfonyl, Trifluormethylsulfonyl, Ethylsulfonyl, Dimethylamino oder Dimethylaminosulfonyl steht,
R⁴ für Wasserstoff, Nitro, Cyano, Fluor, Chlor, Brom, Methyl, Difluormethyl, Trifluormethyl, Dichlormethyl, Trichlormethyl, Chlordifluormethyl, Ethyl, Methoxy, Difluormethoxy, Trifluormethoxy, Chlordifluormethoxy, Ethoxy, Methylthio, Difluormethylthio, Trifluormethylthio, Chlordifluormethylthio, Ethylthio, Methylsulfinyl, Trifluormethylsulfinyl, Ethylsulfinyl, Methylsulfonyl, Trifluormethylsulfonyl, Ethylsulfonyl, Dimethylamino oder Dimethylaminosulfonyl steht, und
R⁵ für Wasserstoff, Methyl, Ethyl, n- oder i-Propyl, n-, i-, s- oder t-Butyl, Cyclopropyl, Cyclopentyl oder Cyclohexyl steht.

5. Verbindungen gemäß einem der Ansprüche 1 bis 4, **dadurch gekennzeichnet, dass** sie als Natrium-, Kalium-, Magnesium-, Calcium-, Ammonium-, C₁-C₄-Alkyl-ammonium-, Di-(C₁-C₄-alkyl)-ammonium-, Tri-(C₁-C₄-alkyl)-ammonium-, Tetra-(C₁-C₄-alkyl)-ammonium, Tri-(C₁-C₄-alkyl)-sulfonium-, C₅-oder C₆-Cycloalkyl-ammonium- oder Di-(C₁-C₂-alkyl)-benzyl-ammonium-Salze oder als Komplexverbindungen mit Metallen vorliegen.

6. Verbindungen gemäß Anspruch 4, **gekennzeichnet durch** die folgenden allgemeinen Formeln (IA), (IB) oder (IC)

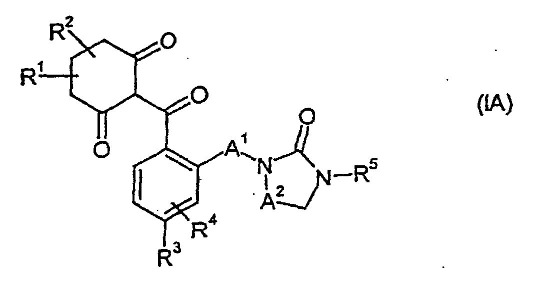

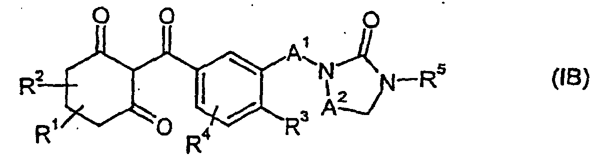

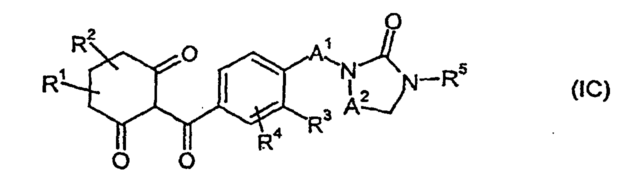

7. Verfahren zum Herstellen von substituierten Benzoylcyclohexandionen gemäß einem der Ansprüche 1 bis 4, **dadurch gekennzeichnet, dass** man 1,3-Cyclohexandion oder dessen Derivate der allgemeinen Formel (II), 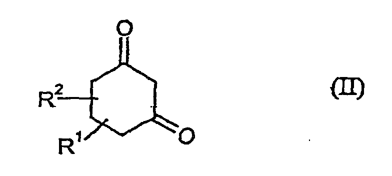 in welcher
R¹ und R² die in einem der Ansprüche 1 bis 4 angegebene Bedeutung haben,
mit substituierten Benzoesäuren der allgemeinen Formel (III), 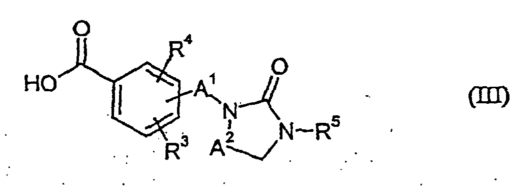 in welcher
A¹, A², R³, R⁴ und R⁵ die in einem der Ansprüche 1 bis 4 angegebene Bedeutung haben,
in Gegenwart eines Dehydratisierungsmittels, gegebenenfalls in Gegenwart eines oder mehrerer Reaktionshilfsmittel und gegebenenfalls in Gegenwart eines Verdünnungsmittels, umsetzt.

8. Verbindungen der Formel (III) 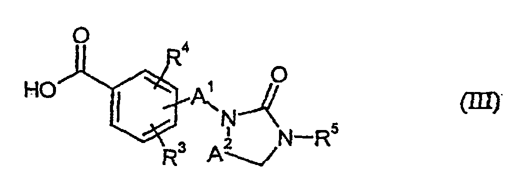 in welcher
A¹, A², R³, R⁴ und R⁵ die in einem der Ansprüche 1 bis 4 angegebene Bedeutung haben.

9. Verbindungen der Formel (IV) 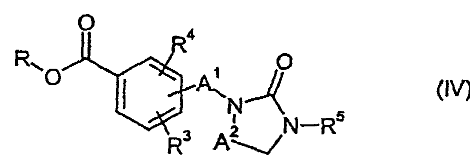 in welcher
R für Alkyl steht und
A¹, A², R³, R⁴ und R⁵ die in einem der Ansprüche 1 bis 4 angegebene Bedeutung haben.

10. Verwendung zumindest einer Verbindung gemäß einem der Ansprüche 1 bis 4 zum Bekämpfen von unerwünschten Pflanzen.

11. Verfahren zum Bekämpfen von unerwünschten Pflanzen, **dadurch gekennzeichnet, dass** man zumindest eine Verbindung gemäß einem der Ansprüche 1 bis 4 auf unerwünschte Pflanzen und/oder ihren Lebensraum einwirken lässt.

12. Mittel, **gekennzeichnet durch** einen Gehalt an mindestens einer Verbindung gemäß einem der Ansprüche 1 bis 4 und üblichen Streckmitteln und/oder oberflächenaktiven Mitteln.

## Claims

1. A substituted benzoylcyclohexanedione of the general formula (I) 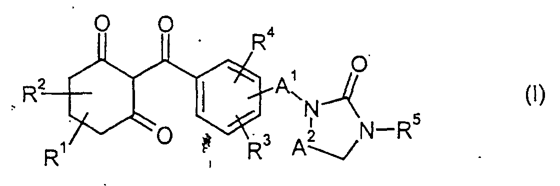 in which
A¹ represents alkanediyl (alkylene) having 1 to 3 carbon atoms,
A² represents alkanediyl (alkylene) having 1 to 3 carbon atoms,
R¹ represents hydrogen, represents phenyl or represents in each case optionally halogen-substituted alkyl, alkylthio or alkoxycarbonyl having in each case 1 to 4 carbon atoms in the alkyl groups,
R² represents hydrogen or represents in each case optionally halogen-substituted alkyl or alkylthio having in each case 1 to 4 carbon atoms, or together with R¹ represents alkanediyl (alkylene) having up to 4 carbon atoms, or - if R¹ and R² are attached to the same carbon atom - together with R¹ and the carbon atom to which R¹ and R² are attached represents a carbonyl grouping (C=O),
R³ represents hydrogen, nitro, cyano, carboxyl, carbamoyl, thiocarbamoyl, halogen, represents in each case optionally halogen-substituted alkyl, alkoxy, alkylthio, alkylsulfinyl, alkylsulfonyl, alkylamino, dialkylamino, alkylsulfonylamino or dialkylaminosulfonyl having in each case 1 to 4 carbon atoms in the alkyl groups, or represents in each case optionally halogen- or C₁-C₄-alkyl-substituted cycloalkylamino, cycloalkylsulfonyl, cycloalkylsulfonylamino or cycloalkylaminosulfonyl having in each case 3 to 6 carbon atoms,
R⁴ represents hydrogen, nitro, cyano, carboxyl, carbamoyl, thiocarbamoyl, halogen, or represents in each case optionally halogen-substituted alkyl, alkoxy, alkylthio, alkylsulfinyl, alkylsulfonyl, alkylamino, dialkylamino or dialkylaminosulfonyl having in each case 1 to 4 carbon atoms in the alkyl groups, and
R⁵ represents hydrogen, represents amino, represents in each case optionally amino-, cyano-, halogen- or C₁-C₄-alkoxy-substituted alkyl, alkoxy, alkylamino, dialkylamino, alkylsulfonyl or alkylsulfonylamino having in each case 1 to 5 carbon atoms in the alkyl groups, represents in each case optionally halogen-substituted alkenyl or alkinyl having in each case up to 5 carbon atoms, represents in each case optionally cyano-, halogen- or C₁-C₄-alkyl-substituted cycloalkyl or cycloalkylalkyl having in each case 3 to 6 carbon atoms in the cycloalkyl group and optionally 1 to 3 carbon atoms in the alkyl moiety, represents cycloalkenyl having 5 or 6 carbon atoms, or represents in each case optionally nitro-, cyano-, carboxyl-, halogen-, C₁-C₄-alkyl-, C₁-C₄-halogenoalkyl-, C₁-C₄-alkoxy- or C₁-C₄-halogenoalkoxy-substituted phenyl, phenyl-C₁-C₄-alkyl, pyridyl or pyrimidinyl,
including all possible tautomeric forms of the compound of the general formula (I) and the possible salts and metal complexes of the compound of the general formula (I),
except for the compound 1-[2,6-dichloro-3-[(2-hydroxy-6-oxo-cyclohexen-1-yl)-carbonyl]-benzyl]-3-methyl-tetrahydro-2(1H)-pyrimidinone.

2. A compound as claimed in claim 1, **characterized in that**
A¹ represents methylene, ethane-1,1-diyl (ethylidene), ethane-1,2-diyl (dimethylene), propane-1,1-diyl, propane-1,2-diyf or propane-1,3-diyl,
A² represents methylene, ethane-1,1-diyl (ethylidene), ethane-1,2-diyl (dimethylene), propane-1,1-diyl, propane-1,2-diyl or propane-1,3-diyl,
R¹ represents hydrogen, represents phenyl or represents in each case optionally fluorine- and/or chlorine-substituted methyl, ethyl, n- or i-propyl, methylthio, ethylthio, n- or i-propylthio, methoxycarbonyl, ethoxycarbonyl, n- or i-propoxycarbonyl,
R² represents hydrogen or represents in each case optionally fluorine- and/or chlorine-substituted methyl, ethyl, n- or i-propyl, methylthio, ethylthio, n- or i-propylthio, or together with R¹ represents methylene, ethane-1,1-diyl, ethane-1,2-diyl (dimethylene), propane-1,1-diyl, propane-1,2-diyl or propane-1,3-diyl, or - if R¹ and R² are attached to the same carbon atom - together with R¹ and the carbon atom to which R¹ and R² are attached represents a carbonyl grouping (C=O),
R³ represents hydrogen, nitro, cyano, carboxyl, carbamoyl, thiocarbamoyl, fluorine, chlorine, bromine, iodine, represents in each case optionally fluorine- and/or chlorine-substituted methyl, ethyl, n- or i-propyl, methoxy, ethoxy, n- or i-propoxy, methylthio, ethylthio, n- or i-propylthio, methylsulfinyl, ethylsulfinyl; n- or i-propylsulfinyl, methylsulfonyl, ethylsulfonyl, n- or i-propylsulfonyl, represents methylamino, ethylamino, n- or i-propylamino, dimethylamino, diethylamino, methylsulfonylamino, ethylsulfonylamino, n- or i-propylsulfonylamino, dimethylaminosulfonyl or diethylaminosulfonyl, or represents in each case optionally fluorine-, chlorine- or methyl-substituted cyclopropylamino, cyclobutylamino, cyclopentylamino, cyclohexylamino, cyclopropylsulfonyl, cyclobutylsulfonyl, cyclopentylsulfonyl, cyclohexylsulfonyl, cyclopropylsulfonylamino, cyclobutylsulfonylamino, cyclopentylsulfonylamino, cyclohexylsulfonylamino, cyclopropylaminosulfonyl, cyclobutylaminosulfonyl, cyclopentylaminosulfonyl or cyclohexylaminosulfonyl,
R⁴ represents hydrogen, nitro, cyano, carboxyl, carbamoyl, thiocarbamoyl, fluorine, chlorine, bromine, iodine, or represents in each case optionally fluorine- and/or chlorine-substituted methyl, ethyl, n- or i-propyl, methoxy, ethoxy, n- or i-propoxy, methylthio, ethylthio, n- or i-propylthio, methylsulfinyl, ethylsulfinyl, n- or i-propylsulfinyl, methylsulfonyl, ethylsulfonyl, n- or i-propylsulfonyl, represents methylamino, ethylamino, n- or i-propylamino, dimethylamino, diethylamino, dimethylaminosulfonyl or diethylaminosulfonyl, and
R⁵ represents hydrogen, represents amino, represents in each case optionally amino-, cyano-, fluorine- and/or chlorine-, methoxy- or ethoxy-substituted methyl, ethyl, n- or i-propyl, n-, i-, s- or t-butyl, methoxy, ethoxy, n- or i-propoxy, n-, i-, s- or t-butoxy, methylamino, ethylamino, n- or i-propylamino, n-, i-, s- or t-butylamino, dimethylamino, diethylamino, dipropylamino, methylsulfonyl, ethylsulfonyl or methylsulfonylamino, represents in each case optionally fluorine-, chlorine- and/or bromine-substituted propenyl, butenyl, propinyl or butinyl, represents in each case optionally cyano-, fluorine-, chlorine-, methyl- or ethyl-substituted cyclopropyl, cyclobutyl, cyclopentyl, cyclohexyl, cyclopropylmethyl, cyclobutylmethyl, cyclopentylmethyl or cyclohexylmethyl, represents cyclohexenyl, or represents in each case optionally fluorine-, chlorine-, bromine-, methyl-, trifluoromethyl-, methoxy-, difluoromethoxy- or trifluoromethoxy-substituted phenyl, benzyl, pyridyl or pyrimidinyl.

3. A compound as claimed in claim 1, **characterized in that**
A¹ represents methylene, ethane-1,2-diyl (dimethylene) or propane-1,3-diyl,
A² represents methylene, ethane-1,2-diyl (dimethylene) or propane-1,3-diyl,
R¹ represents hydrogen, phenyl, methyl, ethyl, n- or i-propyl, methylthio, ethylthio, methoxycarbonyl or ethoxycarbonyl,
R² represents hydrogen, methyl, ethyl, methylthio, ethylthio, or together with R¹ represents methylene, ethane-1,2-diyl (dimethylene) or propane-1,3-diyl (trimethylene), or - if R¹ and R² are attached to the same carbon atom - together with R¹ and the carbon atom to which R¹ and R² are attached represents a carbonyl grouping (C=O),
R³ represents hydrogen, nitro, cyano, fluorine, chlorine, bromine, iodine, represents in each case optionally fluorine- and/or chlorine-substituted methyl, ethyl, methoxy, ethoxy, methylthio, ethylthio, methylsulfinyl, ethylsulfinyl, methylsulfonyl, ethylsulfonyl, represents methylamino, ethylamino, dimethylamino or dimethylaminosulfonyl,
R⁴ represents hydrogen, nitro, cyano, fluorine, chlorine, bromine, iodine, represents in each case optionally fluorine- and/or chlorine-substituted methyl, ethyl, methoxy, ethoxy, methylthio, ethylthio, methylsulfinyl, ethylsulfinyl, methylsulfonyl, ethylsulfonyl, represents methylamino, ethylamino, dimethylamino or dimethylaminosulfonyl, and
R⁵ represents hydrogen, methyl, ethyl, n- or i-propyl, n-, i-, s- or t-butyl, propenyl, propinyl, cyclopropyl, cyclopentyl, cyclohexyl, cyclopropylmethyl, cyclopentylmethyl, cyclohexylmethyl, phenyl or benzyl.

4. A compound as claimed in claim 1, **characterized in that**
A¹ represents methylene or ethane-1,2-diyl (dimethylene),
A² represents methylene or ethane-1,2-diyl (dimethylene),
R¹ represents hydrogen, phenyl, methyl, ethyl, n- or i-propyl, methylthio or ethylthio,
R² represents hydrogen, methyl, ethyl, methylthio, or together with R¹ represents ethane-1,2-diyl or propane-1,3-diyl, or - if R¹ and R² are attached to the same carbon atom - together with R¹ and the carbon atom to which R¹ and R² are attached represents a carbonyl grouping (C=O),
R³ represents hydrogen, nitro, cyano, fluorine, chlorine, bromine, methyl, difluoromethyl, trifluoromethyl, dichloromethyl, trichloromethyl, chlorodifluoromethyl, ethyl, methoxy, difluoromethoxy, trifluoromethoxy, chlorodifluoromethoxy, ethoxy, methylthio, difluoromethylthio, trifluoromethylthio, chlorodifluoromethylthio, ethylthio, methylsulfinyl, trifluoromethylsulfinyl, ethylsulfinyl, methylsulfonyl, trifluoromethylsulfonyl, ethylsulfonyl, dimethylamino or dimethylaminosulfonyl,
R⁴ represents hydrogen, nitro, cyano, fluorine, chlorine, bromine, methyl, difluoromethyl, trifluoromethyl, dichloromethyl, trichloromethyl, chlorodifluoromethyl, ethyl, methoxy, difluoromethoxy, trifluoromethoxy, chlorodifluoromethoxy, ethoxy, methylthio, difluoromethylthio, trifluoromethylthio, chlorodifluoromethylthio, ethylthio, methylsulfinyl, trifluoromethylsulfinyl, ethylsulfinyl, methylsulfonyl, trifluoromethylsulfonyl, ethylsulfonyl, dimethylamino or dimethylaminosulfonyl, and
R⁵ represents hydrogen, methyl, ethyl, n- or i-propyl, n-, i-, s- or t-butyl, cyclopropyl, cyclopentyl or cyclohexyl.

5. A compound as claimed in any of claims 1 to 4, **characterized in that** it is present as sodium, potassium, magnesium, calcium, ammonium, C₁-C₄-alkyl-ammonium, di-(C₁-C₄-alkyl)-ammonium, tri-(C₁-C₄-alkyl)-ammonium, tetra-(C₁-C₄-alkyl)-ammonium, tri-(C₁-C₄-alkyl)-sulfonium, C₅- or C₆-cycloalkyl-ammonium or di-(C₁-C₂-alkyl)-benzyl-ammonium salts or as complex compounds with metals.

6. A compound as claimed in claim 4, **characterized by** the following general formulae (IA), (IB) and (IC)

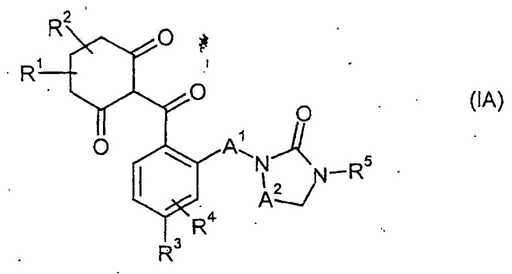

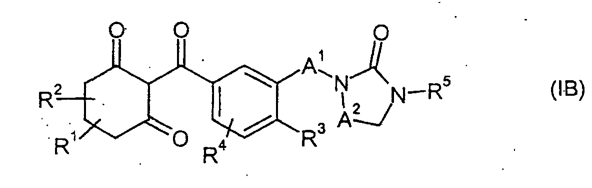

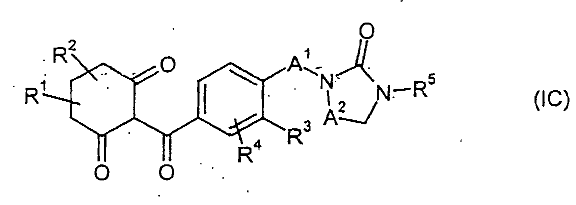

7. A process for preparing a substituted benzoylcyclohexanedione as claimed in any of claims 1 to 4, **characterized in that** 1,3-cyclohexanedione or its derivatives of the general formula (II) 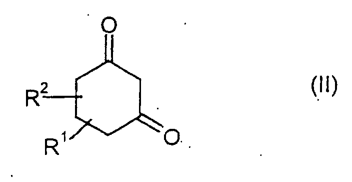 in which
R¹ and R² are each as defined in any of claims 1 to 4,
are reacted with substituted benzoic acids of the general formula (III) 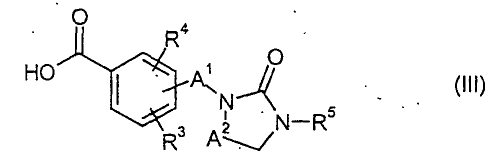 in which
A¹, A², R³, R⁴ and R⁵ are each as defined in any of claims 1 to 4,
in the presence of a dehydrating agent, if appropriate in the presence of one or more reaction auxiliaries and if appropriate in the presence of a diluent.

8. A compound of the formula (III) 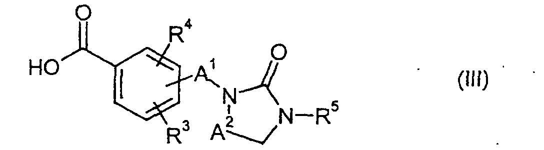 in which
A¹, A², R³, R⁴ and R⁵ are each as defined in any of claims 1 to 4.

9. A compound of the formula (IV) 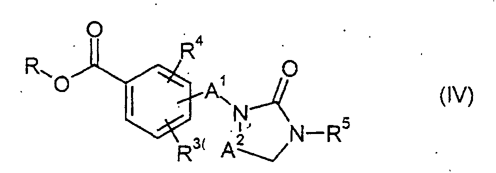 in which
R represents alkyle and
A¹, A², R³, R⁴ and R⁵ are each as defined in any of claims 1 to 4.

10. The use of at least one compound as claimed in any of claims 1 to 4 for controlling undesirable plants.

11. A method for controlling undesirable plants, **characterized in that** at least one compound as claimed in any of claims 1 to 4 is allowed to act on undesirable plants and/or their habitat.

12. A composition, **characterized in that** it comprises at least one compound as claimed in any of claims 1 to 4 and customary extenders and/or surfactants.

## Revendications

1. Benzoylcyclohexanediones substituées de la formule générale (I) : 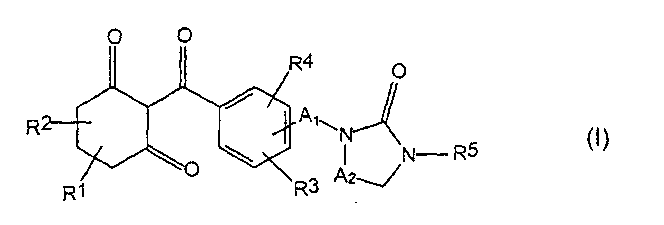 dans laquelle :
A¹ représente un radical alcanediyle (alcoylène) ayant 1 à 3 atomes de carbone ;
A² représente un radical alcanediyle (alcoylène) ayant 1 à 3 atomes de carbone ;
R¹ représente l'atome d'hydrogène, le radical phényle ou un radical alcoyle, alcoylthio ou alcoxycarbonyle le cas échéant substitué par un atome d'halogène, ayant chaque fois 1 à 4 atomes de carbone dans le radical alcoyle ;
R² représente l'atome d'hydrogène ou un radical alcoyle ou àlcoylthio le cas échéant substitué par un atome d'halogène, ayant chaque fois 1 à 4 atomes de carbone, ou représente avec R¹, un radical alcanediyle (alcoylène) ayant jusqu'à 4 atomes de carbone, ou dans le cas où R¹ et R² sont reliés au même atome de carbone, avec R¹ et l'atome de carbone sur lequel R¹ et R² sont reliés, représente un groupement carbonyle (C=O) ;
R³ représente l'atome d'hydrogène, le radical nitro, cyano, carboxy, carbamoyle, thiocarbamoyle, un atome d'halogène, un radical alcoyle, alcoxy, alcoylthio, alcoylsulfinyle, alcoylsulfonyle, alcoylamino, dialcoylamino, alcoylsulfonylamino ou dialcoylsulfonylamino le cas échéant substitué par un atome d'halogène, ayant chaque fois 1 à 4 atomes de carbone dans le radical alcoyle, ou représente un radical cycloalcoylamino, cycloalcoylsulfonyle, cycloalcoylsulfonylamino ou cycloalcoylaminosulfonyle le cas échéant substitué par un atome d'halogène ou un radical alcoyle en C₁-C₄, ayant chaque fois 3 à 6 atomes de carbone ;
R⁴ représente l'atome d'hydrogène, le radical nitro, cyano, carboxy, carbamoyle, thiocarbamoyle, un atome d'halogène, un radical alcoyle, alcoxy, alcoylthio, alcoylsulfinyle, alcoylsulfonyle, alcoylamino, dialcoylamino ou dialcoylaminosulfonyle le cas échéant substitué par un atome d'halogène, ayant chaque fois 1 à 4 atomes de carbone dans le radical alcoyle, et
R⁵ représente l'atome d'hydrogène, représente le radical amino, représente un radical alcoyle, alcoxy, alcoylamino, dialcoylamino, alcoylsulfonyle ou alcoylsulfonylamino le cas échéant substitué par le radical amino, cyano, un atome d'halogène ou un radical alcoxy en C₁-C₄, ayant chaque fois 1 à 5 atomes de carbone dans le radical alcoyle, ou représente un radical alcényle ou alcynyle le cas échéant substitué par un atome d'halogène, ayant chaque fois jusqu'à 5 atomes de carbone, représente un radical cycloalcoyle ou cycloalcoylalcoyle, le cas échéant substitué par le radical cyano, un atome d'halogène ou un radical alcoyle en C₁-C₄, ayant chaque fois 3 à 6 atomes de carbonedans le radical cycloalcoyle et le cas échéant, 1 à 3 atomes de carbone dans la partie alcoyle, représente un radical cycloalcényle ayant 5 ou 6 atomes de carbone, ou représente le radical phényle, phényl(alcoyle en C₁-C₄), pyridinyle ou pyrimidinyle, le cas échéant substitué par le radical nitro, cyano, carboxy, un atome d'halogène, un radical alcoyle en C₁-C₄, halogénoalcoyle en C₁-C₄, alcoxy en C₁-C₄ ou halogénoalcoxy en C₁-C₄ ;
y compris toutes les formes tautomères possibles des composés de la formule générale (I) et les sels et respectivement, les complexes métalliques possibles des composés de la formule générale (I), à l'exception du composé 1-[2,6-dichloro-3-[(2-hydroxy-6-oxocyclohexène-1-yl) carbonyl]benzyl]-3-méthyltétrahydro-2(1H)-pyrimidinone.

2. Composés suivant la revendication 1, **caractérisés en ce que** :
A¹ représente le radical méthylène, éthane-1,1-diyle (éthylidène), éthane-1,2-diyle (diméthylène), propane-1,1-diyle, propane-1,2-diyle ou propane-1,3-diyle,
A² représente le radical méthylène, éthane-1,1-diyle (éthylidène), éthane-1,2-diyle (diméthylène), propane-1,1-diyle, propane-1,2-diyle ou propane-1,3-diyle,
R¹ représente l'atome d'hydrogène, représente le radical phényle ou le radical méthyle, éthyle, n- ou i-propyle, méthylthio, éthylthio, n- ou i-propylthio, méthoxycarbonyle, éthoxycarbonyle, n- ou i-propoxycyclopentylsulfonylamino, cyclohexylsulfonylamino, cyclopropylaminosulfonyle, cyclobutylaminosulfonyle, cyclopentylaminosulfonyle, cyclohexylaminosulfonyle,
R⁴ représente l'atome d'hydrogène, le radical nitro, cyano, carboxy, carbamoyle, thiocarbamoyle, l'atome de fluor, de chlore, de brome, d'iode, représente le radical méthyle, éthyle, n- ou i-propyle, méthoxy, éthoxy, n- ou i-propoxy, méthylthio, éthylthio, n- ou i-propylthio, méthylsulfinyle, éthylsulfinyle, n- ou i-propylsulfinyle, méthylsulfonyle, éthylsulfonyle, n- ou i-propylsulfonyle, le cas échéant substitué par l'atome de fluor et/ou de chlore, représente le radical méthylamino, éthylamino, n- ou i-propylamino, diméthylamino, diéthylamino, diméthylaminosulfonyle ou diéthylaminosulfonyle, et
R⁵ représente l'atome d'hydrogène, le radical amino, représente le radical méthyle, éthyle, n- ou i-propyle, n-, i-, s- ou t-butyle, méthoxy, éthoxy, n-ou i-propoxy, n-, i-, s- ou t-butoxy, méthylamino, éthylamino, n- ou i-propylamino, n-, i-, s- ou t-butylamino, diméthylamino, diéthylamino, dipropylamino, méthylsulfonyle, éthylsulfonyle ou méthylsulfonylamino, le cas échéant substitué par le radical amino, cyano, l'atome de fluor et/ou de chlore, le radical méthoxy ou éthoxy, représente le radical propényle, butényle, propynyle ou butynyle, chaque fois le cas échéant substitué par l'atome de fluor, de chlore et/ou de brome, le radical cyclopropyle, cyclobutyle, cyclopentyle, cyclohexyle, cyclopropylméthyle, cyclobutylméthyle, cyclopentylméthyle ou cyclohexylméthyle, chaque fois le cas échéant substitué par le radical cyano, l'atome de fluor, de chlore, le radical méthyle ou éthyle, représente le radical cyclohexényle, ou représente le radical phényle, benzyle, pyridinyle ou pyrimidinyle, chaque fois le cas échéant substitué par l'atome de fluor, de chlore, de brome, le radical méthyle, trifluorométhyle, méthoxy, difluorométhoxy ou trifluorométhoxy.

3. Composés suivant la revendication 1, **caractérisés en ce que** :
A¹ représente le radical méthylène, éthane-1,2-diyle (diméthylène) ou propane-1,3-diyle,
A² représente le radical méthylène, éthane-1,2-diyle (diméthylène) ou propane-1,3-diyle,
R¹ représente l'atome d'hydrogène, représente le radical phényle, méthyle, éthyle, n- ou i-propyle, méthylthio, éthylthio, méthoxycarbonyle ou éthoxycarbonyle,
R² représente l'atome d'hydrogène, représente le radical méthyle, éthyle, méthylthio, éthylthio, ou représente avec R¹, le radical méthylène, éthane-1,2-diyle (diméthylène) ou propane-1,3-diyle, ou dans le cas où R¹ et R² sont reliés au même atome de carbone, avec R¹ et l'atome de carbone sur lequel R¹ et R² sont reliés, représente un groupement carbonyle (C=O) ;
R³ représente l'atome d'hydrogène, le radical nitro, cyano, l'atome de fluor, de chlore, de brome, d'iode, représente le radical méthyle, éthyle, méthoxy, éthoxy, méthylthio, éthylthio, méthylsulfinyle, éthylsulfinyle, méthylsulfonyle, éthylsulfonyle, le cas échéant substitué par l'atome de fluor et/ou de chlore, représente le radical méthylamino, éthylamino, diméthylamino ou diméthylaminosulfonyle,
R⁴ représente l'atome d'hydrogène, le radical nitro, cyano, l'atome de fluor, de chlore, de brome, d'iode, représente le radical méthyle, éthyle, méthoxy, éthoxy, méthylthio, éthylthio, méthylsulfinyle, éthylsulfinyle, méthylsulfonyle, éthylsulfonyle, le cas échéant substitué par l'atome de fluor et/ou de chlore, représente le radical méthylamino, éthylamino, diméthylamino ou diméthylaminosulfonyle, et
R⁵ représente l'atome d'hydrogène, le radical méthyle, éthyle, n- ou i-propyle, n-, i-, s- ou t-butyle, propényle, propynyle, cyclopropyle, cyclopentyle, cyclohexyle, cyclopropylméthyle, cyclopentylméthyle, cyclohexylméthyle, phényle ou benzyle.

4. Composés suivant la revendication 1, **caractérisés en ce que** :
A¹ représente le radical méthylène ou éthane-1,2-diyle (diméthylène),
A² représente le radical méthylène ou éthane-1,2-diyle (diméthylène),
R¹ représente l'atome d'hydrogène, le radical phényle, méthyle, éthyle, n- ou i-propyle, méthylthio ou éthylthio,
R² représente l'atome d'hydrogène, représente le radical méthyle, éthyle, méthylthio, ou représente avec R¹, le radical éthane-1,2-diyle ou propane-1,3-diyle, ou dans le cas où R¹ et R² sont reliés au même atome de carbone, avec R¹ et l'atome de carbone sur lequel R¹ et R² sont reliés, représente un groupement carbonyle (C=O) ;
R³ représente l'atome d'hydrogène, le radical nitro, cyano, l'atome de fluor, de chlore, de brome, représente le radical méthyle, difluorométhyle, trifluorométhyle, dichlorométhyle, trichlorométhyle, chlorodifluorométhyle, éthyle, méthoxy, difluorométhoxy, trifluorométhoxy, chlorodifluorométhoxy, éthoxy, méthylthio, difluorométhylthio, trifluorométhylthio, chlorodifluorométhylthio, éthylthio, méthylsulfinyle, trifluorométhylsulfinyle, éthylsulfinyle, méthylsulfonyle, trifluorométhylsulfonyle, éthylsulfonyle, diméthylamino ou diméthylaminosulfonyle,
R⁴ représente l'atome d'hydrogène, le radical nitro, cyano, l'atome de fluor, de chlore, de brome, le radical méthyle, difluorométhyle, trifluorométhyle, dichlorométhyle, trichlorométhyle, chlorodifluorométhyle, éthyle, méthoxy, difluorométhoxy, trifluorométhoxy, chlorodifluorométhoxy, éthoxy, méthylthio, difluorométhylthio, trifluorométhylthio, chlorodifluorométhylthio, éthylthio, méthylsulfinyle, trifluorométhylsulfinyle, éthylsulfinyle, méthylsulfonyle, trifluorométhylsulfonyle, éthylsulfonyle, diméthylamino ou diméthylaminosulfonyle, et
R⁵ représente l'atome d'hydrogène, le radical méthyle, éthyle, n- ou i-propyle, n-, i-, s- ou t-butyle, cyclopropyle, cyclopentyle ou cyclohexyle.

5. Composés suivant l'une quelconque des revendications 1 à 4, **caractérisés en ce qu'**ils sont présents sous la forme de sels de sodium, de potassium, de magnésium, de calcium, d'ammonium, de (alcoyl en C₁-C₄)ammonium, de di (alcoyl en C₁-C₄) ammonium, de tri(alcoyl en C₁-C₄)ammonium, de tétra(alcoyl en C₁-C₄) ammonium, de tri (alcoyl en C₁-C₄)sulfonium, de (cycloalcoyl en C₅-C₆)ammonium ou de di (alcoyl en C₁-C₂)benzylammonium,ou sous forme de composés complexes avec des métaux.

6. Composés suivant la revendication 4, **caractérisés par** les formules (IA), (IB) ou (IC) générales suivantes :

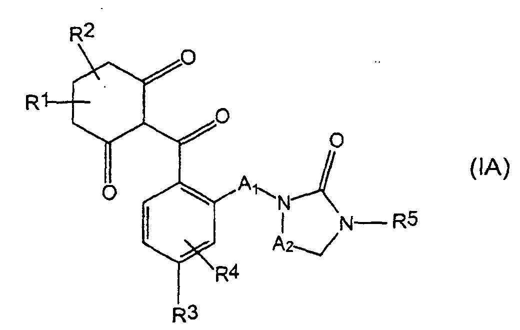

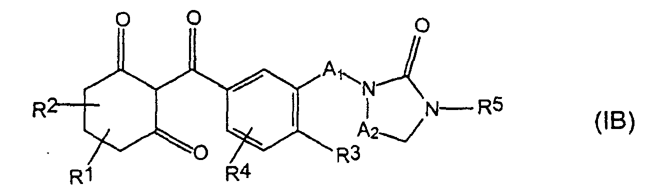

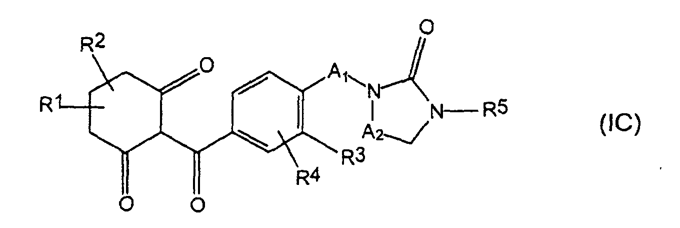

7. Procédé de préparation des benzylcyclohexanediones substituées suivant l'une quelconque des revendications 1 à 4, **caractérisé en ce que** l'on fait réagir la 1,3-cyclohexanedione ou son dérivé de la formule générale (II) : 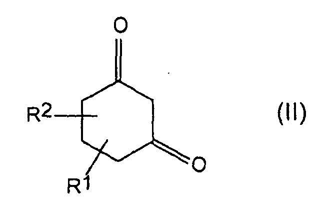 dans laquelle :
R¹ et R² ont les significations indiquées dans l'une quelconque des revendications 1 à 4,
avec des acides benzoïques substitués de la formule générale (III) : 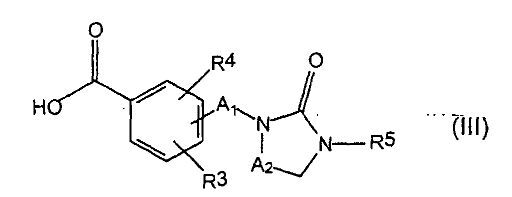 dans laquelle :
A¹, A², R³, R⁴ et R⁵ ont les significations indiquées dans l'une quelconque des revendications 1 à 4,
en présence d'un agent de déshydratation, le cas échéant en présence d'un ou de plusieurs auxiliaires de réaction et le cas échéant, en présence d'un agent de dilution.

8. Composés de la formule (III) : 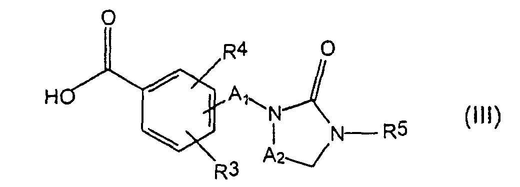 dans laquelle :
A¹, A², R³, R⁴ et R⁵ ont les significations indiquées dans l'une quelconque des revendications 1 à 4.

9. Composés de la formule (IV) : 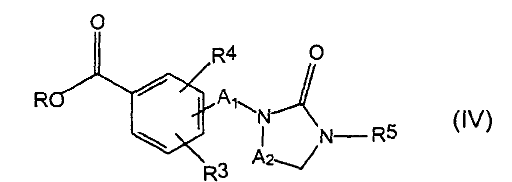 dans laquelle :
R représente un radical alcoyle, et
A¹, A², R³, R⁴ et R⁵ ont les significations indiquées dans l'une quelconque des revendications 1 à 4.

10. Utilisation d'au moins un composé suivant l'une quelconque des revendications 1 à 4, pour lutter contres les végétaux non désirés.

11. Procédé pour lutter contre les végétaux non désirés, **caractérisé en ce que** l'on fait agir au moins un composé suivant l'une quelconque des revendications 1 à 4, sur les végétaux non désirés et/ou leur biotope.

12. Agent, **caractérisé par** une teneur en au moins un composé suivant l'une quelconque des revendications 1 à 4 et des diluants usuels et/ou des agents tensioactifs.
